(19) Europäisches Patentamt
European Patent Office
Office européen des brevets

(11) EP 4 786 595 A1

(12) EUROPEAN PATENT APPLICATION
published in accordance with Art. 153(4) EPC

(43) Date of publication:
**05.08.2026 Bulletin 2026/32**

(21) Application number: **24871079.0**

(22) Date of filing: **29.09.2024**

(51) International Patent Classification (IPC):
***C12N 15/113*** *(2010.01)* ***A61K 31/713*** *(2006.01)*
***A61P 31/20*** *(2006.01)* ***A61P 1/16*** *(2006.01)*

(52) Cooperative Patent Classification (CPC):
**A61K 31/713; A61P 1/16; A61P 31/20; C12N 15/113**

(86) International application number:
**PCT/CN2024/122464**

(87) International publication number:
**WO 2025/067544 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311274541**

(71) Applicant: **Beijing Kawin Technology Share-Holding Co., Ltd.**
**Beijing 100176 (CN)**

(72) Inventors:
- **ZHOU, Desheng**
  **Beijing 100176 (CN)**
- **SHI, Jifeng**
  **Beijing 100176 (CN)**
- **GAO, Jie**
  **Beijing 101318 (CN)**
- **TIAN, Baolei**
  **Beijing 101318 (CN)**
- **WANG, Haojun**
  **Beijing 101318 (CN)**
- **LI, Xiang**
  **Beijing 100176 (CN)**
- **WU, Pengwei**
  **Beijing 100176 (CN)**
- **XIONG, Guoyu**
  **Beijing 100176 (CN)**

(74) Representative: **M. Zardi & Co S.A.**
**Via G. B. Pioda, 6**
**6900 Lugano (CH)**

Remarks:
The complete document including Reference Table(s) and the Sequence Listing(s) can be downloaded from the EPO website

# (54) SIRNA FOR INHIBITING HEPATITIS B VIRUS AND USE THEREOF

(57) Provided are an siRNA for inhibiting hepatitis B virus and the use thereof. Specifically provided is an oligonucleotide for inhibiting HBV or a pharmaceutically acceptable salt thereof. The oligonucleotide comprises an antisense strand, and the antisense strand comprises at least one modified nucleotide, wherein the nucleotide sequence of the antisense strand comprises a sequence or a fragment as set forth in any one of SEQ ID NOs: 3-35. The siRNA has a significant inhibitory effect on HBsAg, HBV DNA and HBeAg produced by cccDNA and HBV DNA integrated into the host genome, both in vivo and in vitro, and has lasting efficacy. Compared with an original sequence, the optimized sequence not only improves the inhibitory effect and the efficacy cycle, but also reduces the off-target toxicity.



Processed by Luminess, 75001 PARIS (FR)

## Description

## CROSS REFERENCE TO RELATED APPLICATION

This application claims priority to Chinese Patent Application No. 2023112745419 filed on September 28, 2023, which is incorporated herein by reference in its entirety.

## TECHNICAL FIELD

The present disclosure belongs to the field of biomedicines, and relates to a small interfering RNA (siRNA) for inhibiting hepatitis B virus (HBV) and a use thereof. Specifically, the present disclosure relates to siRNA with double-stranded modification, and a conjugate and salt thereof. The use is a use in the manufacture of a medicament for treating hepatitis B or chronic infection.

## BACKGROUND

Hepatitis B is a viral disease caused by HBV infection. The main routes of transmission of hepatitis B comprise blood-borne transmission, sexual transmission and mother-to-child transmission. The World Health Organization (WHO) estimates that approximately 250 million people suffer from chronic HBV infection worldwide. After years of infection, about 15% to 40% of patients with chronic HBV infection develop severe adverse outcomes, such as liver cirrhosis, liver failure, and hepatocellular carcinoma. Currently, nearly one million patients die from HBV-related complications each year (Nicolini LA, et al. Int. J. Environ. Res. Public Health 2019, 16, 3307). The basis of chronic HBV infection is the persistent presence of covalently closed circular DNA (cccDNA) of HBV in infected hepatocytes to serve as a reservoir and template for viral replication. Moreover, as a byproduct of viral replication, HBV DNA can be randomly integrated into the host genome. Although the integrated HBV sequences cannot sustain viral replication, they can continuously produce viral proteins, such as hepatitis B surface antigen (HBsAg) and transcriptionally regulated HBV x protein (Suarez AAR, et al. Liver International. 2021; 41, Suppl. 1: 15-23). The existing therapeutic drugs for hepatitis B are relatively limited, and mainly comprise nucleoside analogs (NUCs) such as entecavir and tenofovir, and pegylated interferon-$\alpha$ (Peg-IFN-$\alpha$). However, these therapeutic drugs often struggle to eliminate the viruses, and HBsAg loss remains rare. Therefore, the aforementioned therapeutic drugs require long-term administration to maintain viral suppression and prevent viral relapse after discontinuation.

The cost of lifelong treatment or intervention imposes a heavy economic burden on patients. Therefore, there is an urgent need to develop new therapeutic strategies to achieve a "functional cure" for chronic hepatitis B (CHB) infection, which is defined as sustained HBsAg loss, undetectable HBV DNA, normal liver enzymes, and improved liver histopathology after drug discontinuation. HBsAg loss is an indicator for profound suppression of HBV replication and the sole indicator for safe treatment discontinuation. Therefore, CHB represents an unmet clinical need, and continuous efforts are required to develop novel molecules, combination therapies, and entirely innovative treatment strategies to achieve the goal of HBV elimination.

In 1998, American scientists Andrew Fire and Craig Mello discovered the novel biological mechanism that small RNA molecules can mediate the degradation of specific mRNAs (Fire, Andrew, et al. nature 391.6669 (1998): 806-811). When RNA molecules are present in a double-stranded form in cells, this biological mechanism is triggered to cause the phenomenon of RNA interference. This discovery heralded the beginning of a new field of research. These two scientists were accordingly awarded the 2006 Nobel Prize in Physiology or Medicine. When double-stranded RNA (dsRNA) binds to the protein complex Dicer, Dicer cleaves the dsRNA into fragments, which are siRNAs. Subsequently, another protein complex, RNA-induced silencing complex (RISC), binds to these fragments. As a result, one strand of an siRNA duplex is removed, while the other strand remains associated with the RISC. Guided by single-stranded RNA, RISC recognizes and degrades mRNA of a target gene to inhibit the expression of a specific protein, thereby leading to targeted gene silencing.

RNA interference has opened up a new field for the application of genetic technologies. dsRNA molecules have been artificially designed to silence specific genes in humans, animals, or plants. These artificially designed and synthesized dsRNA molecules (siRNAs) for gene silencing can be introduced into cells to activate the RNA interference mechanism for degrading the corresponding mRNAs. Currently, this approach serves as a vital research tool in biology and biomedicine. Further, numerous siRNA drugs have been developed to treat viral infections, cardiovascular diseases, cancer, endocrine disorders, and various other diseases. Most siRNA therapies, whether in the research and development phase or already approved for marketing, have exhibited excellent therapeutic effects. Since the first siRNA drug was launched in 2018, at least four siRNA drugs have been approved for marketing in either the European Union or the United States. Therefore, the inhibition of the expression of specific target genes by RNA interference techniques has become an effective therapeutic approach for diseases.

There have been HBV siRNA drugs in the clinical stage that exhibit specified effects in inhibiting HBsAg, HBV

DNA, and HBeAg. However, issues regarding the toxicity and safety of HBV siRNA drugs remain significant and cannot be overlooked. Therefore, there is an urgent need to develop HBV siRNA drugs that offer both excellent efficacy and high safety.

## SUMMARY

In order to solve the problems in the prior art, an objective of the present disclosure is to provide siRNA for inhibiting HBV and a use thereof. The siRNA exhibits significant long-lasting inhibitory effects on HBsAg, HBV DNA, and hepatitis B e antigen (HBeAg) produced from cccDNA and host genome-integrated HBV DNA both *in vivo* and *in vitro*. Compared with the original sequence, the optimized siRNA not only enhances the inhibitory effect and the duration of efficacy, but also reduces off-target toxicity.

In one aspect, the present disclosure provides an oligonucleotide for inhibiting HBV or a pharmaceutically acceptable salt thereof, wherein the oligonucleotide comprises a sense strand (SS) and an antisense strand (AS); the nucleotide sequence of the SS comprises a sequence shown in SEQ ID NO: 1 or a fragment thereof; and the nucleotide sequence of the AS comprises a sequence shown in SEQ ID NO: 3 or a fragment thereof.

In another aspect, the present disclosure provides a composition, comprising the oligonucleotide or the pharmaceutically acceptable salt thereof as described above, and optionally a pharmaceutically acceptable carrier.

In another aspect, the present disclosure provides a use of the oligonucleotide or the pharmaceutically acceptable salt thereof and/or the composition as described above in the manufacture of a medicament for reducing at least one of HBsAg, HBeAg, and HBV DNA in serum of a subject.

In another aspect, the present disclosure provides a use of the oligonucleotide or the pharmaceutically acceptable salt thereof and/or the composition in the manufacture of a medicament for treating HBV infection or hepatitis B, and HBV-hepatitis D virus (HDV) coinfection.

In another aspect, the present disclosure provides a method for reducing at least one of HBsAg, HBeAg, and HBV DNA in serum, comprising administering a therapeutically effective amount of oligonucleotide or the pharmaceutically acceptable salt thereof as described above to a subject.

In another aspect, the present disclosure provides a method for treating HBV infection or hepatitis B, comprising administering a therapeutically effective amount of oligonucleotide or pharmaceutically acceptable salt thereof as described above to a subject.

## BRIEF DESCRIPTION OF THE DRAWINGS

FIG. 1 shows the synthesis process of siRNA.

FIG. 2 shows inhibitory effects of compounds at different concentrations on serum HBsAg, HBeAg, and HBV DNA in HBV transgenic mice after single subcutaneous administration.

FIG. 3 shows inhibitory effects of compounds AL0107045, AL0107048, AL0107049, AL0107050, AL0107051, AL0107052, AL0107053, and AL0107054 on serum HBV DNA, HBeAg, and HBsAg in HBV transgenic mice after single subcutaneous administration.

FIG. 4 shows inhibitory effects of compounds L0107045, AL0107057, and AL0107058 on serum HBV DNA, HBeAg, and HBsAg in HBV transgenic mice after single subcutaneous administration.

FIG. 5 shows the curves of the effects of different compounds on body weight changes of male and female SD rats treated with different compounds.

FIG. 6 shows the effects of different compounds on an expression level of glutamic-oxaloacetic transaminase (GOT) in male and female SD rats.

## DETAILED DESCRIPTION OF THE EMBODIMENTS

In the present disclosure, unless otherwise specified, the scientific and technical terms used herein have the meanings commonly understood by those skilled in the art. The terms and laboratory procedures related to protein and nucleic acid chemistry, molecular biology, cell and tissue cultivation, microbiology, and immunology herein are all widely-used terms and routine procedures in respective fields. Moreover, in order to well understand the present disclosure, the definitions and explanations of related terms are provided below.

As used herein, the term "about" or "approximately" refers to a value close to a stated reference value. In some embodiments, unless otherwise specified or apparent from the context, the term "about" or "approximately" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less of the reference value in either direction (greater or smaller) (except that such a number would exceed

100% of a possible value).

As used herein, "hepatitis B virus" or "HBV" refers to a virus of the family *Hepadnaviridae*. HBV is a small (such as 3.2 kb) hepatotropic DNA virus that encodes four open reading frames and seven proteins. The seven proteins encoded by HBV comprise small (S), medium (M), and large (L) surface antigens (HBsAg) or envelope (Env) proteins, a pre-core protein, a core protein, a viral polymerase (Pol), and an HBx protein. HBV expresses three surface antigens or envelope proteins L, M, and S, where S is the smallest and L is the largest. Extra domains in the M and L proteins are designated Pre-S2 and Pre-S1, respectively. The core protein is a subunit of a viral nucleocapsid. The Pol is required for the synthesis of viral DNA (reverse transcriptase, RNaseH, and primer), which occurs within the nucleocapsid localized in the cytoplasm of infected hepatocytes. The pre-core is a core protein with an N-terminal signal peptide. Before being secreted by infected cells, the pre-core is proteolytically processed at N and C termini to produce the so-called HBeAg. The HBx protein is required for the efficient transcription of cccDNA. The HBx protein is not a viral structural protein. With the exception of the core and the polymerase, which share an mRNA, all viral proteins of HBV have respective mRNAs. With the exception of the pre-core protein, the HBV proteins do not undergo post-translational proteolytic processing.

As used herein, the terms "HBV antigen", "antigenic polypeptide of HBV", "HBV antigenic polypeptide", "antigenic protein of HBV", "immunogenic polypeptide of HBV", and "HBV immunogen" all refer to a polypeptide capable of inducing an immune response against HBV in a subject, such as humoral and/or cell-mediated response. The HBV antigen may be an HBV polypeptide or a fragment or epitope thereof, or a combination of a plurality of HBV polypeptides or a portion or derivative thereof. The HBV antigen can elicit a protective immune response in a host, for example, inducing an immune response against a viral disease or infection and/or producing an immunity against a viral disease or infection in a subject (namely, vaccination), which protects the subject from the viral disease or infection. For example, the HBV antigen may comprise a polypeptide from any HBV protein, or an immunogenic fragment thereof, for example, HBeAg, pre-core protein, HBsAg (S, M, or L protein), core protein, viral polymerase, or HBx protein derived from any HBV genotype (including genotypes A, B, C, D, E, F, G, and/or H, or a combination thereof).

As used herein, each of the terms "HBV core antigen", "HBcAg", and "core antigen" refers to an HBV antigen capable of inducing an immune response against the HBV core protein in a subject, such as a humoral and/or cell-mediated response. Each of the terms "core", "core polypeptide", and "core protein" refers to the HBV core protein. The full-length core antigen of HBV has a length typically of 183 amino acids and comprises an assembly domain (1st to 149th amino acids) and a nucleic acid-binding domain (150th to 183rd amino acids). A 34-residue nucleic acid-binding domain is essential for pregenomic RNA encapsidation. The 34-residue nucleic acid-binding domain also serves as a nuclear import signal. The 34-residue nucleic acid-binding domain comprises 17 arginine residues and is highly basic, which is consistent with the function of this domain. The HBV core protein is dimeric in a solution. Dimers of the HBV core protein self-assemble into an icosahedral capsid. Each dimer of the HBV core protein comprises a four-$\alpha$-helix bundle flanked by $\alpha$-helical domains at two ends. A truncated HBV core protein lacking the nucleic acid-binding domain can also produce a capsid.

As used herein, the term "HBV-associated disease" or "HBV-related disease" refers to a disease or condition caused by or associated with HBV infection or replication. The term "HBV-related disease" comprises a disease, disorder, or condition that would benefit from a reduction in HBV gene expression or replication. Non-limiting examples of the HBV-related disease comprise, for example, acute hepatitis B, acute fulminant hepatitis B, CHB, liver fibrosis, end-stage liver disease, and hepatocellular carcinoma.

As used herein, the term "complementary" refers to a structural relationship between nucleotides (for example, between two nucleotides on opposite nucleic acids or on opposite regions of a single nucleic acid strand) that permits base pairing between the nucleotides. For example, the purine nucleotide of one nucleic acid and a pyrimidine nucleotide of an opposite nucleic acid that is complementary to the purine nucleotide can be base-paired by forming a hydrogen bond. In some embodiments, complementary nucleotides can be base-paired in a Watson-Crick manner or in any other manner allowing for the formation of a stable duplex. In some embodiments, two nucleic acids can have nucleotide sequences that are complementary to each other, so as to form a complementary region, as described herein.

As used herein, the term "siRNA off-target effects (OTEs)" means that, due to non-specificity during siRNA action, siRNA may affect genes other than a target gene, resulting in silencing of non-target genes. The characteristics and delivery manner of siRNA can both lead to a decline in specificity, thereby causing OTEs. OTEs associated with siRNAs are classified into the following three main types: miRNA-like OTEs, immune stimulation, and saturation of RNAi machinery.

As used herein, the term "strand" refers to a single continuous sequence of nucleotides linked together by internucleotide linkages (such as phosphodiester linkages and phosphorothioate linkages). In some embodiments, the strand has two free termini, such as a 5'-terminus and a 3'- terminus.

As used herein, the term "deoxyribonucleotide" refers to a nucleotide with hydrogen at the 2' position of pentose in contrast to a ribonucleotide. A modified deoxyribonucleotide is a deoxyribonucleotide with a modification or substitution (including a modification or substitution in a sugar, a phosphate group, or a base or a modification or substitution of the sugar, the phosphate group, or the base) of one or more atoms at a position other than the 2' position.

As used herein, the term "double-stranded oligonucleotide" refers to an oligonucleotide that is substantially in a

duplex form. In some embodiments, one or more duplex regions of the double-stranded oligonucleotide are formed by complementary base pairing between antiparallel sequences of nucleotides in covalently separated nucleic acid strands. In some embodiments, one or more duplex regions of the double-stranded oligonucleotide are formed by complementary base pairing between antiparallel sequences of nucleotides in covalently linked nucleic acid strands. In some embodiments, when one or more duplex regions of the double-stranded oligonucleotide are formed from a single nucleic acid strand, the single nucleic acid strand is folded (for example, through a hairpin) to provide complementary, antiparallel sequences of nucleotides that can be base-paired. In some embodiments, the double-stranded oligonucleotide comprises two covalently separated nucleic acid strands that are fully duplexed with each other. However, in some embodiments, the double-stranded oligonucleotide comprises two covalently separated nucleic acid strands that are partially duplexed, for example, there is an overhang at one or two termini. In some embodiments, the double-stranded oligonucleotide comprises antiparallel sequences of nucleotides that are partially complementary to each other, and thus may have one or more mismatches. The one or more mismatches may comprise internal mismatches or terminal mismatches.

As used herein, the term "oligonucleotide" refers to a short nucleic acid, such as a short nucleic acid with a length of less than 100 nucleotides. The oligonucleotide may comprise ribonucleotides, deoxyribonucleotides, and/or modified nucleotides, including, for example, modified ribonucleotides. The oligonucleotide may be single-stranded or double-stranded. The oligonucleotide may or may not have a duplex region. As a set of non-limiting examples, the oligonucleotide may be, but is not limited to, siRNA, microRNA (miRNA), short hairpin RNA (shRNA), Dicer substrate interfering RNA (dsiRNA), an antisense oligonucleotide, short siRNA, or single-stranded siRNA. In some embodiments, a double-stranded oligonucleotide is an RNAi oligonucleotide.

As used herein, the term "nucleotide analog" refers to a non-standard nucleotide, comprising a non-naturally occurring ribonucleotide or deoxyribonucleotide. Exemplary nucleotide analogs may be modified at any position to alter some chemical properties of these nucleotide analogs while still retaining the ability of these nucleotide analogs to exert intended functions. The nucleotide analogs herein can serve as nucleotide monomers to replace nucleotides on a backbone. Specifically, the nucleotide analogs include, but are not limited to: 2'-methoxyethyl (moe)-modified nucleotides, 2'-methoxy (m)-modified nucleotides, 2'-deoxy-2'-fluoro (f)-modified nucleotides, phosphorothioate (s)-modified nucleotides, adenosine-2' phosphate (A-2'-5')-modified nucleotides, uridine-2' phosphate (U-2'-5')-modified nucleotides, guanosine-2' phosphate (G-2'-5')-modified nucleotides, guanosine-glycol nucleic acid (Ggn)-modified nucleotides, adenosine-glycol nucleic acid (Agn)-modified nucleotides, N-(2,3-dihydroxypropyl)-3,5-bis(trifluoromethyl)benzamide (GNF-BX)-modified nucleotides, or 5'-phosphate mimic-modified nucleotides.

As used herein, the term "AGO2 protein" refers to Argonaute 2 protein, also known as eukaryotic translation initiation factor 2C subunit 2 (EIF2C2). The AGO2 protein comprises four globular domains of N-terminal, PAZ, MID, and PIWI domains, and two linker domains L1 and L2. The AGO2 protein is a member of the Argonaute (AGO) protein family. The AGO2 protein is widely expressed in organisms and is a core component of RISC. The AGO2 protein possesses endoribonuclease activity. The AGO2 protein can inhibit the expression of a target gene by promoting the maturation of miRNA and regulating the biosynthesis and function of miRNA, thereby playing a key role in various pathophysiological processes. It is known that the 5'-phosphate group can enhance the interaction between some nucleic acid inhibitor molecules and the AGO2 protein.

As used herein, (Agn) refers to adenosine-glycol nucleic acid (GNA), (Cgn) refers to cytidine-glycol nucleic acid (GNA), (Ggn) refers to guanosine-glycol nucleic acid (GNA), and (Tgn) refers to thymidine-glycol nucleic acid (GNA).

As used herein, the term "modified sequence of nucleotides", "modified sequence of AS and fragment thereof", "modified sequence of SS or fragment thereof", or "modified sequence" refers to a modified nucleotide sequence produced by chemically modifying a nucleic acid to enhance the functional delivery of the nucleic acid to a target cell or reduce potential toxic and side effects of the nucleic acid, for example, to increase the stability of the nucleic acid against a nuclease and to improve the target-binding affinity of the nucleic acid. The modified oligonucleotide herein may comprise one or more chemically modified ribonucleotides in either or both of the AS and the SS. The modification may be a modification on a nucleotide, a modification between nucleotides, or a substitution with a nucleotide analog. The modification on a nucleotide can be a chemical modification on a base, a ribose, and/or a phosphate group. The chemical modification herein comprises a modification on a nucleotide, a modification between nucleotides, or a substitution with a nucleotide analog. The modification herein is selected from at least one of a 2'-methoxyethyl (moe) modification, a 2'-methoxy (m) modification, a 2'-deoxy-2'-fluoro (f) modification, a phosphorothioate group (s) modification, an adenosine-2' phosphate (A-2'-5') modification, an uridine-2' phosphate (U-2'-5') modification, a guanosine-2' phosphate (G-2'-5') modification, a guanosine-glycol nucleic acid (Ggn) modification, an adenosine-glycol nucleic acid (Agn) modification, an uridine-glycol nucleic acid (Ugn) modification, an N-(2,3-dihydroxypropyl)-3,5-bis(trifluoromethyl)benzamide (GNF-BX) modification, a 5'-vinylphosphonic acid (Vp) modification, or a 5'-vinylphosphonate-2'-N-acetyl modification. Specifically, the ribose modification comprises any one or more of a 2'-methoxyethyl (moe) modification, a 2'-methoxy (m) modification, an N-(2,3-dihydroxypropyl)-3,5-bis(trifluoromethyl)benzamide (GNF-BX) modification, a 5'-vinylphosphonic acid (Vp) modification, a 2'-deoxy-2'-fluoro (f) modification, and a 5'-vinylphosphonate-2'-N-acetyl modification. The modification between nucleotides comprises a modification of a phosphodiester linkage in a phosphate

group, such as phosphorothioate(s) modification. The phosphorothioate modification is achieved by substituting at least one oxygen atom in the phosphodiester linkage with a sulfur atom. Herein, regarding the substitution with a nucleotide analog, for example, an adenosine analog at a corresponding position can be substituted with adenosine-2’ phosphate (A-2’-5’), a uridine analog at a corresponding position can be substituted with uridine-2’ phosphate (U-2’-5’), a guanosine analog at a corresponding position can be substituted with guanosine-2’ phosphate (G-2’-5’), a guanosine analog at a corresponding position can be substituted with guanosine-glycol nucleic acid (Ggn), an adenosine analog at a corresponding position can be substituted with adenosine-glycol nucleic acid (Agn), a uridine analog at a corresponding position can be substituted with uridine-glycol nucleic acid (Ugn), and a uridine analog at a corresponding position can be substituted with 5’-vinylphosphonate-2’-N-acetyl-uridine (APU001). The corresponding substitutions at specific positions of nucleotide analogs in the present disclosure are shown in Tables 1 and 2 of the present disclosure.

As used herein, the term "inhibition" indicates a reduction of gene expression in a cell, cell population, or tissue when treated with the siRNA, siRNA conjugate, or composition described in the present disclosure compared to the cell, cell population, or tissue without such a treatment. The term "inhibition" can be used interchangeably with "reduction", "silencing", "downregulation", "suppression", and other similar terms, and comprises any level of inhibition. Preferably, the inhibition comprises statistically significant inhibition or clinically significant inhibition.

As used herein, the term "pharmaceutical composition" refers to a mixture or solution comprising a therapeutically effective amount of an active pharmaceutical ingredient and a pharmaceutical excipient to be administered together with the active pharmaceutical ingredient to mammals in need thereof (such as humans).

As used herein, the term "subject" means any animal, preferably mammals, and more preferably human, which are to undergo or have undergone treatment according to a method in an embodiment of the present disclosure. The term "mammal" as used herein encompasses any mammal. Examples of the mammal include, but are not limited to, cattle, horses, sheep, pigs, cats, dogs, mice, rats, rabbits, guinea pigs, non-human primates (NHP) such as monkeys or apes, and humans, and preferably comprise humans.

The term "therapeutically effective amount" refers to an amount of a compound or molecule of the present disclosure that can (i) treat or prevent a particular disease, disorder, or condition, (ii) attenuate, ameliorate, or eliminate one or more symptoms of a particular disease, disorder, or condition, or (iii) prevent or delay the onset of one or more symptoms of the particular disease, disorder, or condition described herein when administered to a subject. The therapeutically effective amount depends on the compound, a state of a disease to be treated, a severity of a disease to be treated, an age and a relative health status of a subject, a route and form of administration, determination of an attending physician or veterinarian, and other factors.

In one aspect, the present disclosure provides an oligonucleotide for inhibiting HBV or a pharmaceutically acceptable salt thereof. The oligonucleotide comprises SS and AS. The nucleotide sequence of the SS comprises a sequence shown in SEQ ID NO: 1 or a fragment thereof. The nucleotide sequence of the AS comprises a sequence shown in SEQ ID NO: 3 or a fragment thereof.

In some embodiments, the AS is further subjected to a modification, and the modification is selected from one or more of a 2’-methoxyethyl modification, a 2’-methoxy (m) modification, a 2’-deoxy-2’-fluoro (f) modification, a phosphorothioate group (s) modification, an adenosine-2’ phosphate (A-2’-5’) modification, a uridine-2’ phosphate (U-2’-5’) modification, a guanosine-2’ phosphate (G-2’-5’) modification, a guanosine-glycol nucleic acid (Ggn) modification, an adenosine-glycol nucleic acid (Agn) modification, an N-(2,3-dihydroxypropyl)-3,5-bis(trifluoromethyl)benzamide (GNF-BX) modification, or a 5’-phosphate mimic modification.

In some embodiments, the modification is selected from one or more of a 2’-methoxyethyl modification, an adenosine-2’ phosphate (A-2’-5’) modification, a uridine-2’ phosphate (U-2’-5’) modification, a guanosine-2’ phosphate (G-2’-5’) modification, a guanosine-glycol nucleic acid (Ggn) modification, an adenosine-glycol nucleic acid (Agn) modification, an N-(2,3-dihydroxypropyl)-3,5-bis(trifluoromethyl)benzamide (GNF-BX) modification, or a 5’-phosphate mimic modification.

In some embodiments, any of nucleotides at positions 1 to 8 starting from the 5’ terminus of the AS is correspondingly modified.

In some embodiments, the modified AS comprises a nucleotide sequence shown in any of SEQ ID NOs: 4-35 or a fragment thereof.

In some embodiments, the modification comprises a modification on a nucleotide, a modification between nucleotides, or a nucleotide analog substitution modification.

In some embodiments, the modification is selected from one or more of a 2’-methoxyethyl modification, a 2’-methoxy (m) modification, a 2’-deoxy-2’-fluoro (f) modification, a phosphorothioate group (s) modification, an adenosine-2’ phosphate (A-2’-5’) modification, a uridine-2’ phosphate (U-2’-5’) modification, a guanosine-2’ phosphate (G-2’-5’) modification, a guanosine-glycol nucleic acid (Ggn) modification, an adenosine-glycol nucleic acid (Agn) modification, a uridine-glycol nucleic acid (Ugn) modification, an N-(2,3-dihydroxypropyl)-3,5-bis(trifluoromethyl)benzamide (GNF-BX) modification, or a 5’-phosphate mimic modification.

In some embodiments, the ribose modification comprises any one or more of a 2’-methoxyethyl (moe)

modification, a 2'-methoxy (m) modification, an N-(2,3-dihydroxypropyl)-3,5-bis(trifluoromethyl)benzamide (GNF-BX) modification, a 5'-vinylphosphonic acid (Vp) modification, a 2'-deoxy-2'-fluoro (f) modification, and a 5'-vinylphosphonate-2'-N-acetyl modification.

In some embodiments, the base modification includes, but is not limited to, a 5'-vinylphosphonate modification or a 5'-vinylphosphonate-2'-N-acetyl modification. When the two modifications are employed, they occur at a 5'-terminal nucleotide of the AS.

In some embodiments, the modification between nucleotides comprises a modification of a phosphodiester linkage in a phosphate group, such as phosphorothioate(s) modification. The phosphorothioate modification is achieved by substituting at least one oxygen atom in the phosphodiester linkage with a sulfur atom.

In some embodiments, the 5'-phosphate mimic is 5'-oxymethylphosphonate, 5'-vinylphosphonic acid (Vp), 5'-vinylphosphonate-2'-N-acetyl, or 5'-malonylphosphonate; and preferably, the 5'-vinylphosphonic acid (Vp) or the 5'-vinylphosphonate-2'-N-acetyl.

In some embodiments, the 5'-phosphate mimic modification is located at the 5'-terminus of the AS, and preferably, the 5'-phosphate mimic is the 5'-vinylphosphonic acid (Vp).

In an exemplary embodiment, a compound produced after the 5'-terminal nucleotide of the AS of the oligonucleotide is modified with Vp has the following structural formula:

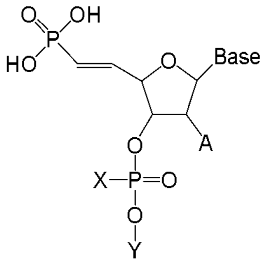

X is -SH or -OH,

Base represents a pyrimidine or purine base, Y represents other nucleosides on the oligonucleotide, and A is selected from any one of substituents conventionally selected in the art, such as halogen (such as F), OH, $OCH_3$, $OCF_3$, $OCH_2CH_3$, 2'-methoxyethyl, and 2'-N-acetyl.

In some embodiments, a nucleotide modified with the 5'-phosphate mimic is 5'-vinylphosphonate-2'-methoxyuridine.

In some embodiments, a nucleotide modified with the 5'-phosphate mimic is 5'-vinylphosphonate-2'-N-acetyluridine (APU001).

In some embodiments, all the nucleotides of the SS and the AS are modified nucleotides.

In some embodiments, the oligonucleotide comprises at least one modified internucleotide linkage.

In some embodiments, the at least one modified internucleotide linkage is a phosphorothioate linkage.

In some embodiments, a 5'-terminal phosphate group of the oligonucleotide can enhance an interaction between the oligonucleotide and the Argonaut 2 protein. However, oligonucleotides with a 5'-phosphate group are generally susceptible to degradation by phosphatases or other enzymes, which limits the bioavailability *in vivo.* In some embodiments, the oligonucleotide comprises an analog of a 5'-phosphate group that can effectively protect the oligonucleotide from degradation by phosphatases or other enzymes, which increases the cellular uptake of the oligonucleotide or improves the pharmacokinetic properties of the oligonucleotide.

In some embodiments, 4'-carbon of a pentose of the 5'-terminal nucleotide of the AS comprises a phosphate analog modification in which a phosphate analog replaces $-CH_2OH$ linked to the 4'-carbon of the pentose.

In some embodiments, the phosphate analog is oxymethylphosphonate, vinylphosphonate, or malonylphosphonate.

In the present disclosure, the terms "phosphate mimic", "phosphate analog", and "phosphonate" can be used interchangeably and all refer to a phosphate group in which at least one O is substituted with C. An exemplary structure is as follows:

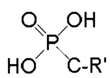,

wherein R' is independently selected from the group consisting of H, optionally substituted alkyl, optionally substituted alkenyl, optionally substituted alkynyl, etc.

In some embodiments, the phosphate analog is vinylphosphonate, wherein a vinyl group can be linked to the 4'-carbon of the pentose of the oligonucleotide by replacing the $-CH_2OH$ originally on the pentose. An exemplary structure is as follows:

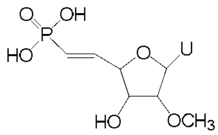

wherein U represents a uracil base.

In some embodiments, the phosphate analog is oxymethylphosphonate, wherein an oxygen atom of an oxymethyl group is linked to the 4'-carbon of the sugar of the oligonucleotide. In some embodiments, the oxymethylphosphonate is represented by $-O-CH_2-PO(OH)_2$ or $-O-CH_2-PO(OR)_2$, wherein R is independently selected from the group consisting of H, $CH_3$, alkyl, $CH_2CH_2CN$, $CH_2OCOC(CH_3)_3$, $CH_2OCH_2CH_2Si(CH_3)_3$, and a protective group. In some embodiments, the alkyl is $CH_2CH_3$. Generally, R is independently selected from the group consisting of H, $CH_3$, and $CH_2CH_3$.

In some embodiments, the modification refers to a substitution, such as a substitution in the nucleotide sequence shown in SEQ ID NO: 3 of the AS. In some embodiments, 2'-methoxy-modified uridine "Um" at the 5' terminus of the AS is substituted with a mononucleotide "APU001". In some embodiments, a 2'-methoxy-modified nucleotide "Gm" in the AS of the oligonucleotide is substituted with a mononucleotide "Ggn". In some embodiments, a 2'-deoxy-2'-fluoro-modified nucleotide "Gf" or "Gfs" in the AS of the oligonucleotide is substituted with the mononucleotide "Ggn". In some embodiments, a 2'-methoxy-modified nucleotide "Um" in the AS of the oligonucleotide is substituted with a mononucleotide "Ugn". In some embodiments, a 2'-deoxy-2'-fluoro-modified nucleotide "Uf" in the AS of the oligonucleotide is substituted with the mononucleotide "Ugn". In some embodiments, a 2'-methoxy-modified nucleotide "Am" in the AS of the oligonucleotide is substituted with a mononucleotide "Agn". In some embodiments, a 2'-deoxy-2'-fluoro-modified nucleotide "Af" in the AS of the oligonucleotide is substituted with the mononucleotide "Agn". In some embodiments, the 2'-methoxy-modified nucleotide "Um" in the AS of the oligonucleotide is substituted with a mononucleotide "U-2'-5'". In some embodiments, the 2'-deoxy-2'-fluoro-modified nucleotide "Uf" in the AS of the oligonucleotide is substituted with the mononucleotide "U-2'-5'". In some embodiments, the 2'-methoxy-modified nucleotide "Am" in the AS of the oligonucleotide is substituted with a mononucleotide "A-2'-5'". In some embodiments, the 2'-deoxy-2'-fluoro-modified nucleotide "Af" in the AS of the oligonucleotide is substituted with the mononucleotide "A-2'-5'". In some embodiments, the 2'-methoxy-modified nucleotide "Gm" in the AS of the oligonucleotide is substituted with a mononucleotide "G-2'-5'". In some embodiments, the 2'-deoxy-2'-fluoro-modified nucleotide "Gf" in the AS of the oligonucleotide is substituted with the mononucleotide "G-2'-5'". In some embodiments, the 2'-methoxy-modified nucleotide "Am" in the AS of the oligonucleotide is substituted with a mononucleotide "GNF-BX". In some embodiments, the 2'-deoxy-2'-fluoro-modified nucleotide "Af" in the AS of the oligonucleotide is substituted with the mononucleotide "GNF-BX". In some embodiments, the 2'-methoxy-modified nucleotide "Um" in the AS of the oligonucleotide is substituted with the mononucleotide "GNF-BX". In some embodiments, the 2'-methoxy-modified nucleotide "Gm" in the AS of the oligonucleotide is substituted with the mononucleotide "GNF-BX". In some embodiments, a 3'-phosphorothioate group- and 2'-deoxy-2'-fluoro-modified nucleotide "Gfs" in the AS of the oligonucleotide is substituted with a mononucleotide "G(moe)". In some embodiments, the 2'-methoxy-modified nucleotide "Gm" in the AS of the oligonucleotide is substituted with the mononucleotide "G(moe)". In some embodiments, the 2'-deoxy-2'-fluoro-modified nucleotide "Gf" in the AS of the oligonucleotide is substituted with the mononucleotide "G(moe)". In some embodiments, the 2'-methoxy-modified nucleotide "Um" in the AS of the oligonucleotide is substituted with a mononucleotide "U(moe)". In some embodiments, the 2'-methoxy-modified nucleotide "Am" in the AS of the oligonucleotide is substituted with a mononucleotide "A(moe)". In some embodiments, the 2'-deoxy-2'-fluoro-modified nucleotide "Af" in the AS of the oligonucleotide is substituted with the mononucleotide "A(moe)".

In some embodiments, the substitution preferably occurs at any of nucleotides at positions 1 to 8 starting from the 5' terminus of the AS. In a more preferred embodiment, APU001 is a uridine analog to substitute the 5'-terminal nucleotide of the AS, and A-2'-5', U-2'-5', G-2'-5', Ggn, Agn, Ugn, and GNF-BX optionally substitute the corresponding nucleotides at the positions 2 to 8 starting from the 5' terminus of the AS.

In the present disclosure, the detailed information of specific substitution manners and positions of nucleotides can be found in Tables 1 and 2.

In some embodiments, the oligonucleotide is an oligonucleotide with a chain length of 19 to 21 base pairs, for example, a chain length of 19, 20, or 21 base pairs. The oligonucleotide comprises SS and AS that have a same number or different numbers of base pairs.

In some embodiments, the SS has a chain length of 19 bases. In some embodiments, the AS has a chain length of 21 bases.

In some embodiments, the oligonucleotide comprises a combination of SS and AS selected from the group consisting of:

(1) the SS shown in SEQ ID NO: 1 and the AS shown in SEQ ID NO: 3;
(2) the SS shown in SEQ ID NO: 1 and the AS shown in SEQ ID NO: 4;
(3) the SS shown in SEQ ID NO: 1 and the AS shown in SEQ ID NO: 5;
(4) the SS shown in SEQ ID NO: 1 and the AS shown in SEQ ID NO: 6;
(5) the SS shown in SEQ ID NO: 1 and the AS shown in SEQ ID NO: 7;
(6) the SS shown in SEQ ID NO: 1 and the AS shown in SEQ ID NO: 8;
(7) the SS shown in SEQ ID NO: 1 and the AS shown in SEQ ID NO: 9;
(8) the SS shown in SEQ ID NO: 1 and the AS shown in SEQ ID NO: 10;
(9) the SS shown in SEQ ID NO: 1 and the AS shown in SEQ ID NO: 11;
(10) the SS shown in SEQ ID NO: 1 and the AS shown in SEQ ID NO: 12;
(11) the SS shown in SEQ ID NO: 1 and the AS shown in SEQ ID NO: 13;
(12) the SS shown in SEQ ID NO: 1 and the AS shown in SEQ ID NO: 14;
(13) the SS shown in SEQ ID NO: 1 and the AS shown in SEQ ID NO: 15;
(14) the SS shown in SEQ ID NO: 1 and the AS shown in SEQ ID NO: 16;
(15) the SS shown in SEQ ID NO: 1 and the AS shown in SEQ ID NO: 17;
(16) the SS shown in SEQ ID NO: 1 and the AS shown in SEQ ID NO: 18;
(17) the SS shown in SEQ ID NO: 1 and the AS shown in SEQ ID NO: 19;
(18) the SS shown in SEQ ID NO: 1 and the AS shown in SEQ ID NO: 20;
(19) the SS shown in SEQ ID NO: 1 and the AS shown in SEQ ID NO: 21;
(20) the SS shown in SEQ ID NO: 1 and the AS shown in SEQ ID NO: 22;
(21) the SS shown in SEQ ID NO: 1 and the AS shown in SEQ ID NO: 23;
(22) the SS shown in SEQ ID NO: 1 and the AS shown in SEQ ID NO: 24;
(23) the SS shown in SEQ ID NO: 1 and the AS shown in SEQ ID NO: 25;
(24) the SS shown in SEQ ID NO: 1 and the AS shown in SEQ ID NO: 26;
(25) the SS shown in SEQ ID NO: 1 and the AS shown in SEQ ID NO: 27;
(26) the SS shown in SEQ ID NO: 1 and the AS shown in SEQ ID NO: 28;
(27) the SS shown in SEQ ID NO: 1 and the AS shown in SEQ ID NO: 29;
(28) the SS shown in SEQ ID NO: 1 and the AS shown in SEQ ID NO: 30;
(29) the SS shown in SEQ ID NO: 1 and the AS shown in SEQ ID NO: 31;
(30) the SS shown in SEQ ID NO: 1 and the AS shown in SEQ ID NO: 32;
(31) the SS shown in SEQ ID NO: 2 and the AS shown in SEQ ID NO: 33;
(32) the SS shown in SEQ ID NO: 1 and the AS shown in SEQ ID NO: 34; and
(33) the SS shown in SEQ ID NO: 1 and the AS shown in SEQ ID NO: 35.

In some embodiments, the oligonucleotide comprises a combination of SS and AS selected from the group consisting of:

(1) the SS shown in SEQ ID NO: 1 and the AS shown in SEQ ID NO: 5;
(2) the SS shown in SEQ ID NO: 1 and the AS shown in SEQ ID NO: 6;
(3) the SS shown in SEQ ID NO: 1 and the AS shown in SEQ ID NO: 7;
(4) the SS shown in SEQ ID NO: 1 and the AS shown in SEQ ID NO: 8;
(5) the SS shown in SEQ ID NO: 1 and the AS shown in SEQ ID NO: 9;
(6) the SS shown in SEQ ID NO: 1 and the AS shown in SEQ ID NO: 10;
(7) the SS shown in SEQ ID NO: 1 and the AS shown in SEQ ID NO: 11;
(8) the SS shown in SEQ ID NO: 1 and the AS shown in SEQ ID NO: 12;
(9) the SS shown in SEQ ID NO: 1 and the AS shown in SEQ ID NO: 13;
(10) the SS shown in SEQ ID NO: 1 and the AS shown in SEQ ID NO: 14;
(11) the SS shown in SEQ ID NO: 1 and the AS shown in SEQ ID NO: 15;
(12) the SS shown in SEQ ID NO: 1 and the AS shown in SEQ ID NO: 16;
(13) the SS shown in SEQ ID NO: 1 and the AS shown in SEQ ID NO: 17;
(14) the SS shown in SEQ ID NO: 1 and the AS shown in SEQ ID NO: 18;
(15) the SS shown in SEQ ID NO: 1 and the AS shown in SEQ ID NO: 19;
(16) the SS shown in SEQ ID NO: 1 and the AS shown in SEQ ID NO: 20;
(17) the SS shown in SEQ ID NO: 1 and the AS shown in SEQ ID NO: 21;
(18) the SS shown in SEQ ID NO: 1 and the AS shown in SEQ ID NO: 22;
(19) the SS shown in SEQ ID NO: 1 and the AS shown in SEQ ID NO: 23;
(20) the SS shown in SEQ ID NO: 1 and the AS shown in SEQ ID NO: 24;
(21) the SS shown in SEQ ID NO: 1 and the AS shown in SEQ ID NO: 25;

(22) the SS shown in SEQ ID NO: 1 and the AS shown in SEQ ID NO: 26;
(23) the SS shown in SEQ ID NO: 1 and the AS shown in SEQ ID NO: 27;
(24) the SS shown in SEQ ID NO: 1 and the AS shown in SEQ ID NO: 28;
(25) the SS shown in SEQ ID NO: 1 and the AS shown in SEQ ID NO: 29;
(26) the SS shown in SEQ ID NO: 1 and the AS shown in SEQ ID NO: 30;
(27) the SS shown in SEQ ID NO: 1 and the AS shown in SEQ ID NO: 31;
(28) the SS shown in SEQ ID NO: 1 and the AS shown in SEQ ID NO: 32;
(29) the SS shown in SEQ ID NO: 2 and the AS shown in SEQ ID NO: 33;
(30) the SS shown in SEQ ID NO: 1 and the AS shown in SEQ ID NO: 34; and
(31) the SS shown in SEQ ID NO: 1 and the AS shown in SEQ ID NO: 35.

In some embodiments, at least one nucleotide of the oligonucleotide is conjugated to one or more targeting ligands.

The targeting ligand can be any ligand capable of targeting a specific receptor. Examples of the targeting ligand comprise a folate, N-acetylgalactosamine (GalNAc), galactose, mannose, mannose-6P, a sugar cluster (such as a GalNAc cluster, a mannose cluster, or a galactose cluster), or an aptamer. A cluster is a combination of two or more sugar units. These targeting ligands also comprise an integrin receptor ligand, a chemokine receptor ligand, transferrin, biotin, a serotonin receptor ligand, prostate-specific membrane antigen (PSMA), endothelin, glutamate carboxypeptidase II (GCPII), somatostatin, a low-density lipoprotein (LDL) receptor ligand, and a high-density lipoprotein (HDL) receptor ligand. These ligands can also be based on a nucleic acid, such as an aptamer. The aptamer can be unmodified or have any combination of modifications disclosed herein.

In some embodiments, the targeting ligand is conjugated to the 3' terminus of the SS of the oligonucleotide.

In some embodiments, the targeting ligand comprises a carbohydrate, an amino sugar, cholesterol, a polypeptide, or a lipid.

In some embodiments, the targeting ligand comprises the amino sugar.

In some embodiments, the targeting ligand comprises a GalNAc moiety. GalNAc is covalently conjugated to a phosphate group of a 3'-terminal nucleotide of the SS of the oligonucleotide in a trivalent state to produce an oligonucleotide sequence with a GalNAc carrier.

In some embodiments, the GalNAc moiety is a monovalent GalNAc moiety, a divalent GalNAc moiety, a trivalent GalNAc moiety, or a tetravalent GalNAc moiety.

In some embodiments, the targeting ligand has the following structure:

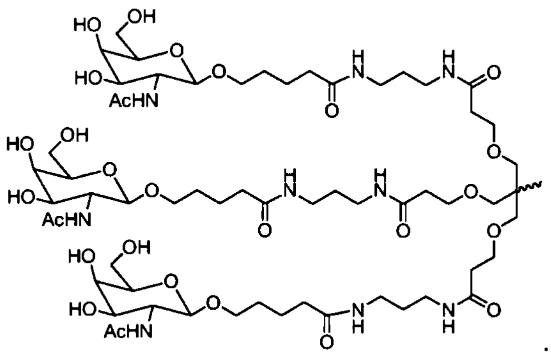

.

In some embodiments, the targeting ligand is conjugated to the 3' terminus of the SS of the oligonucleotide through a linker to produce the following conjugate:

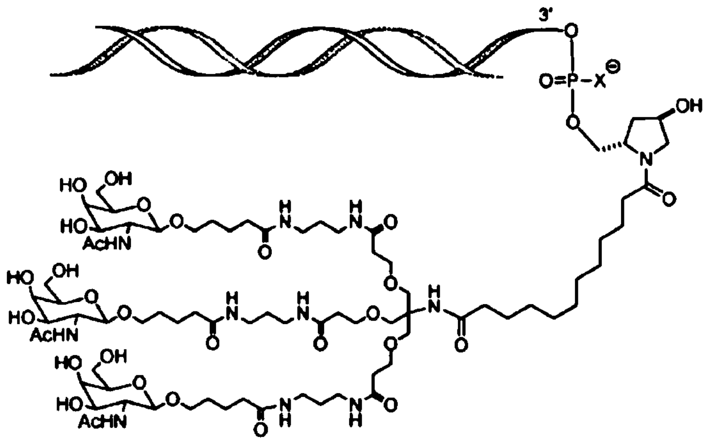

,

wherein X is selected from S or O.

In some embodiments, the oligonucleotide comprises a combination of SS and AS selected from the group consisting of:

(1) the SS shown in SEQ ID NO: 36 and the AS shown in SEQ ID NO: 3;
(2) the SS shown in SEQ ID NO: 36 and the AS shown in SEQ ID NO: 4;
(3) the SS shown in SEQ ID NO: 36 and the AS shown in SEQ ID NO: 27;
(4) the SS shown in SEQ ID NO: 36 and the AS shown in SEQ ID NO: 28;
(5) the SS shown in SEQ ID NO: 36 and the AS shown in SEQ ID NO: 29;
(6) the SS shown in SEQ ID NO: 36 and the AS shown in SEQ ID NO: 30;
(7) the SS shown in SEQ ID NO: 36 and the AS shown in SEQ ID NO: 31;
(8) the SS shown in SEQ ID NO: 36 and the AS shown in SEQ ID NO: 32;
(9) the SS shown in SEQ ID NO: 37 and the AS shown in SEQ ID NO: 39;
(10) the SS shown in SEQ ID NO: 38 and the AS shown in SEQ ID NO: 33;
(11) the SS shown in SEQ ID NO: 36 and the AS shown in SEQ ID NO: 34; and
(12) the SS shown in SEQ ID NO: 36 and the AS shown in SEQ ID NO: 35.

In some embodiments, the oligonucleotide comprises a combination of SS and AS selected from the group consisting of:

(1) the SS shown in SEQ ID NO: 36 and the AS shown in SEQ ID NO: 27;
(2) the SS shown in SEQ ID NO: 36 and the AS shown in SEQ ID NO: 28;
(3) the SS shown in SEQ ID NO: 36 and the AS shown in SEQ ID NO: 29;
(4) the SS shown in SEQ ID NO: 36 and the AS shown in SEQ ID NO: 30;
(5) the SS shown in SEQ ID NO: 36 and the AS shown in SEQ ID NO: 31;
(6) the SS shown in SEQ ID NO: 36 and the AS shown in SEQ ID NO: 32;
(7) the SS shown in SEQ ID NO: 37 and the AS shown in SEQ ID NO: 39;
(8) the SS shown in SEQ ID NO: 38 and the AS shown in SEQ ID NO: 33;
(9) the SS shown in SEQ ID NO: 36 and the AS shown in SEQ ID NO: 34; and
(10) the SS shown in SEQ ID NO: 36 and the AS shown in SEQ ID NO: 35.

In another aspect, the present disclosure provides a composition, comprising the oligonucleotide or the pharmaceutically acceptable salt thereof as described above, and optionally a pharmaceutically acceptable carrier.

In some embodiments, a dosage form of the composition is an oral preparation, an intravenous injection, a subcutaneous injection, or an intramuscular injection, preferably the subcutaneous injection.

In some embodiments, the composition further comprises an additional drug for treating HBV infection. Preferably, the additional drug is a nucleoside analog, an interferon antiviral drug, an HBV entry inhibitor, a capsid assembly inhibitor, an HBsAg secretion inhibitor, an anti-HBsAg antibody, a hepatitis B therapeutic vaccine, a host immunomodulator, an apoptosis protein antagonist, etc.

In another aspect, the present disclosure provides a use of the oligonucleotide or the pharmaceutically acceptable salt thereof as described above or the composition as described above in the manufacture of a medicament for reducing at least one of HBsAg, HBeAg, and HBV DNA in serum of a subject.

In another aspect, the present disclosure provides a use of the oligonucleotide or the pharmaceutically

acceptable salt thereof as described above or the composition as described above in the manufacture of a medicament for treating HBV infection or hepatitis B and/or HBV-HDV coinfection.

In some embodiments, the HBV infection is chronic HBV infection.

In some embodiments, the hepatitis B is CHB.

In another aspect, the present disclosure provides a method for treating HBV infection or hepatitis B and/or HBV-HDV coinfection, comprising the following step: administrating to a subject in need thereof a therapeutically effective amount of the oligonucleotide or the pharmaceutically acceptable salt thereof as described or the composition as described above.

In some embodiments, the HBV infection is chronic HBV infection.

In some embodiments, the hepatitis B is CHB.

In another aspect, the present disclosure provides the oligonucleotide or the pharmaceutically acceptable salt thereof as described above or the composition as described above, for use in the treatment of HBV infection or hepatitis B and/or HBV-HDV coinfection.

In some embodiments, the HBV infection is chronic HBV infection.

In some embodiments, the hepatitis B is CHB. Various formulations have been developed to facilitate the use of the oligonucleotide. For example, the oligonucleotide can be delivered to a subject or a cellular environment through a formulation that minimizes degradation, promotes delivery and/or uptake, or provides an additional beneficial characteristic for the oligonucleotide in the formulation. In some embodiments, the siRNA comprising the oligonucleotide (such as a single-stranded or double-stranded oligonucleotide) for reducing HBsAg, HBeAg, and HBV DNA or a composition thereof is provided herein. The siRNA or the composition thereof can be properly formulated such that, when the siRNA or the composition thereof is administered to a subject (either administered to a direct environment of a target cell or administered systemically), a sufficient amount of the oligonucleotide can enter cells to reduce HBsAg, HBeAg, and HBV DNA. Any of various appropriate oligonucleotide formulations can be used to deliver the oligonucleotide for reducing HBsAg, HBeAg, and HBV DNA, as disclosed herein. In some embodiments, the oligonucleotide is formulated in a buffer solution, such as phosphate buffered saline (PBS), a liposome, a micellar structure, and a capsule. In some embodiments, the naked oligonucleotide or conjugate thereof is formulated in water or an aqueous solution (such as pH-adjusted water). In some embodiments, the naked oligonucleotide or conjugate thereof is formulated in an alkaline buffer aqueous solution (such as PBS).

An oligonucleotide formulation including a cationic lipid can be used to facilitate transfection of the oligonucleotide into a cell. For example, the cationic lipid can comprise, for example, lipofectin, a cationic glycerol derivative, and a polycationic molecule (such as polylysine).

Therefore, in some embodiments, the formulation comprises a lipid nanoparticle. In some embodiments, the excipient comprises a liposome, a lipid, a lipid complex, a microsphere, a microparticle, a nanosphere, or a nanoparticle, or may be otherwise formulated for administration to a cell, a tissue, an organ, or a body of a subject in need thereof.

In some embodiments, the formulation disclosed herein comprises an excipient. In some embodiments, the excipient confers to the composition enhanced stability, absorption, solubility, and/or efficacy of the active ingredient. In some embodiments, the excipient is a buffering agent (such as sodium citrate, sodium phosphate, tris base, or sodium hydroxide) or a vehicle (such as a buffer solution, petrolatum, dimethyl sulfoxide, or a mineral oil). In some embodiments, the oligonucleotide is lyophilized to extend its shelf life, and prepared into a solution before use (such as, administration to a subject). Therefore, an excipient in a composition including any of the oligonucleotides described herein can be a lyoprotectant (such as mannitol, lactose, polyethylene glycol, or polyvinylpyrrolidone) or a collapse temperature modifier (such as dextran, ficoll, or gelatin).

In some embodiments, the pharmaceutical composition is formulated into a dosage form that is compatible with an intended administration route. Examples of the administration route comprise parenteral administration, such as intravenous, intradermal, subcutaneous, oral (such as inhalation), transdermal (topical), transmucosal, and rectal administration. Typically, the administration route is intravenous or subcutaneous administration.

Pharmaceutical compositions suitable for injectable use comprise sterile aqueous solutions (where water-soluble) or dispersions and sterile powders for the extemporaneous preparation of sterile injectable solutions or dispersions. For intravenous or subcutaneous administration, a suitable carrier comprises normal saline, bacteriostatic water, Cremophor EL™ (BASF, Parsippany, N.J.), or PBS. The carrier can be a solvent or a dispersion medium, comprising, for example, water, ethanol, a polyol (such as glycerol, propylene glycol, and liquid polyethylene glycol), and a suitable mixture thereof. In many cases, preferably, the composition comprises an isotonic agent, for example, a sugar, a polyol such as mannitol and sorbitol, and sodium chloride. A sterile injectable solution can be prepared as follows: a desired amount of oligonucleotide and one or a combination of the required components listed above are added to a selected solvent, and filter sterilization is then conducted.

In some embodiments, the composition may comprise at least about 0.1% or more of therapeutic agent (such as an oligonucleotide for reducing HBsAg, HBeAg, and HBV DNA), and a percentage of a weight or volume of one or more active ingredients may be about 1% to about 80% or more of a weight or volume of the total composition. Those skilled in

the field of preparing such pharmaceutical formulations will consider factors such as solubility, bioavailability, biological half-life, administration route, shelf life of a product, and other pharmacological considerations, and thus various dosages and treatment regimens may be desirable.

Even though many embodiments involve liver-targeted delivery of any oligonucleotide disclosed herein, targeting of other tissues can also be considered.

In some embodiments, administration of the oligonucleotide described herein results in a reduction in levels of HBsAg, HBeAg, and HBV DNA. In some embodiments, the reduction in the levels of HBsAg, HBeAg, and HBV DNA can be a reduction to 1% or less, 5% or less, 10% or less, 15% or less, 20% or less, 25% or less, 30% or less, 35% or less, 40% or less, 45% or less, 50% or less, 55% or less, 60% or less, 70% or less, 80% or less, or 90% or less compared to appropriate control levels. The appropriate control levels can be levels of HBsAg, HBeAg, and HBV DNA in serum of a subject that is not administered with the oligonucleotide described herein. In some embodiments, an effect of delivering the oligonucleotide to a cell by the method disclosed herein is evaluated after a limited period of time. For example, levels of HBsAg, HBeAg, and HBV DNA in serum can be analyzed at least 8 h, 12 h, 18 h, or 24 h or at least 1 d, 2 d, 3 d, 4 d, 5 d, 6 d, 7 d, or 14 d after the oligonucleotide is delivered to the cell.

In some embodiments, the oligonucleotide is delivered in a form of a transgene that is engineered to express the oligonucleotide disclosed herein (for example, in a form of shRNA) in a cell. In some embodiments, any oligonucleotide disclosed herein is delivered using a transgene engineered to express the oligonucleotide. The transgene can be delivered using a viral vector (such as an adenovirus, a retrovirus, a vaccinia virus, a poxvirus, adeno-associated virus, or herpes simplex virus) or a non-viral vector (such as a plasmid or synthetic mRNA). In some embodiments, the transgene can be injected directly into a subject.

This aspect of the present disclosure relates to a method for treating HBV infection, hepatitis B, and/or one or more symptoms or complications thereof in a subject by reducing HBsAg, HBeAg, and HBV DNA. In some embodiments, the method may comprise administering an effective amount of any of the oligonucleotides disclosed herein to a subject in need. In some embodiments, such a treatment can be used, for example, to reduce or prevent hypercholesterolemia (high level of LDL-cholesterol), atherosclerosis, coronary heart disease (such as coronary artery disease), angina pectoris, shortness of breath, sweating, nausea, dizziness, shortness of breath, arrhythmia, palpitation, stroke (that is, the death of brain cells due to insufficient blood and oxygen flows to the brain), weakness, confusion, difficulty speaking, dizziness, difficulty walking or standing upright, blurred vision, numbness in the face, arms, and legs, severe headache, loss of consciousness, peripheral artery disease, and/or kidney problems (such as chronic kidney disease). In some embodiments, such a treatment can be used, for example, to treat or prevent hypercholesterolemia, atherosclerosis, and/or one or more symptoms or complications associated therewith.

Accordingly, in some embodiments, the present disclosure provides a method for treating a subject at risk of (or susceptible to) hypercholesterolemia, atherosclerosis, and/or one or more symptoms or complications thereof (including coronary heart disease (such as coronary artery disease), angina pectoris, shortness of breath, sweating, nausea, dizziness, shortness of breath, arrhythmia, palpitation, stroke (that is, the death of brain cells due to insufficient blood and oxygen flows to the brain), weakness, confusion, difficulty speaking, dizziness, difficulty walking or standing upright, blurred vision, numbness in the face, arms, and legs, severe headache, loss of consciousness, peripheral artery disease, and/or kidney problems (such as chronic kidney disease)).

In some aspects, the present disclosure provides a method for preventing the disease, disorder, symptom, or condition described herein in a subject by administering a therapeutic agent (such as the oligonucleotide or a vector or transgene encoding the same) to the subject. In some embodiments, the subject to be treated refers to an individual who would therapeutically benefit from, for example, reductions in amounts of HBsAg, HBeAg, and HBV DNA in serum.

The method described herein generally involves administering an effective amount (namely, an amount capable of causing a desired therapeutic result) of oligonucleotide to the subject. A therapeutically acceptable amount can be an amount capable of treating a disease or disorder. An appropriate dosage for any subject will depend on some factors, including a body size, a body surface area, and an age of the subject, a particular composition to be administered, one or more active ingredients in the composition, a time and route of administration, general health, and other drugs to be administered concurrently.

In some embodiments, any of the compositions disclosed herein is administered to a subject enterally (for example, oral administration, through a gastric feeding tube, through a duodenal feeding tube, through gastrostomy, or rectal administration), parenterally (for example, subcutaneous injection, intravenous injection or infusion, intra-arterial injection or infusion, or intramuscular injection), topically (for example, epicutaneous administration, inhalation, through eye drops, or through mucous membranes), or by direct injection into a target organ (for example, a liver of the subject). Generally, the oligonucleotide disclosed herein is administered intravenously or subcutaneously.

In some embodiments, the oligonucleotide is administered at a dose ranging from 0.1 mg/kg to 25 mg/kg (for example, 1 mg/kg to 9 mg/kg). In some embodiments, the oligonucleotide is administered at a dose ranging from 0.1 mg/kg to 9 mg/kg or from 0.5 mg/kg to 9 mg/kg.

As a set of non-limiting examples, the oligonucleotide of the present disclosure is generally administered once

yearly, twice yearly, quarterly (once every three months), bimonthly (once every two months), monthly, once half a month, or weekly.

In some embodiments, the subject to be treated is human (such as a human patient) or a non-human primate or other mammalian subject. Other exemplary subjects comprise domestic animals such as dogs and cats; livestock such as horses, cattle, pigs, sheep, goats, and chickens; and animals such as mice, rats, guinea pigs, and hamsters.

For the purpose of clear and concise description, features are described herein as part of the same or separate embodiments. However, it will be understood that the scope of the present disclosure may comprise some embodiments with combinations of all or some of the features described.

The present disclosure will be described in detail below with reference to specific examples. However, the examples are provided for illustrative purposes only and are not intended to limit the present disclosure.

## Examples

### Example 1: Design of siRNA

Candidate oligonucleotide sequences complementary to an HBV genotype D (subtype ayw, V01460 J02203) were generated using a computer-based algorithm. Some of the sequences were also complementary to genotypes A, B, and C or had no more than 2 mismatches with the genotypes A, B, and C. The siRNA was designed as a double-stranded structure with SS and AS of 19/20 pairing, and the AS had two overhangs complementary to an mRNA sequence. In some complementary pairing sequences, a base at position 1 and/or position 19 of the 5' terminus of SS was substituted with a different base from HBV mRNA, and a base at a corresponding position of AS was altered accordingly to form pairing with SS. In some sequences, a base at an individual position within SS was substituted to create a mismatch with a corresponding position of AS. In some sequences, a ribonucleotide at an individual position was substituted with a deoxyribonucleotide. In some sequences, U, A, or G at an individual position in AS was substituted with hypoxanthine (I) to create wobble base pairing with SS. Table 1 lists the designed siRNA-modified oligonucleotide sequences. Table 2 lists the modified oligonucleotide sequences optimized based on AL0105138.

Table 1. Modified oligonucleotides

| Modified sequence No. | Sense 5'-3' | Sequence ID No. | Anti-sense 5'-3' | Sequence ID No. |
|---|---|---|---|---|
| AL01051 25 | GmsCmsCmGmGmAmCfCmGfUfGfUmGmCmAmCmUmUmCm | 40 | GmsAfsAmGmUmGfCmAfCmAmCmGfGmUfCmCfGmGfCmsAmsGm | 47 |
| AL01051 27 | UmsGmsUmGmCmAmCfUmUfCfGfCmUmUmCmAmCmCmAm | 41 | UmsGfsGmUmGmAfAmGfCmGmAmAfGmUfGmCfAmCfAmsGmsGm | 48 |
| AL01051 28 | CmsCmsGmUmGmUmGfCmAfCfUfUmCmGmCmUmUmCmAm | 42 | UmsGfsAmAmGmCfGmAfAmGmUmGfCmAfCmAfCmGfGmsUmsCm | 49 |
| AL01051 31 | GmsAmsCmCmGmUmGfUmGfCfAfCmUmUmCmGmCmUmAm | 43 | UmsAfsGmCmGmAfAmGfUmGmCmAfCmAfCmGfImUfCmsCmsGm | 50 |
| AL01051 32 | GmsAmsCmCmGmUmGfUmGfCmAfCmUmUmCmGmCmUmAm | 44 | UmsAfsGmCmGmAfAmGfUmGmCmAfCmAfCmGfImUfCmsCmsGm | 50 |

(continued)

| Modified sequence No. | Sense 5'-3' | Sequence ID No. | Anti-sense 5'-3' | Sequence ID No. |
|---|---|---|---|---|
| AL01051 33 | GmsAmsCmCmGmUmGfUmGfCfAmCmUmUmCmGmCmUmAm | 45 | UmsAfsGmCmGmAfAmGfUmGmCmAfCmAfCmGfImUfCmsCmsGm | 50 |
| AL01051 34 | GmsAmsCmCmGmUmGfUmGmCfAfCmUmUmCmGmCmUmAm | 46 | UmsAfsGmCmGmAfAmGfUmGmCmAfCmAfCmGfImUfCmsCmsGm | 50 |
| AL01051 37 | GmsAmsCmCmGmUmGfUmGfCfAmCmUmUmCmGmCmUmAm | 45 | VpUmAfsGmCmGmAfAmGfUmGmCmAfCmAfCmGfImUfCmsCmsGm | 51 |
| AL01051 38 | UmsGmsUmGmCmAmCfUmCfCfGmCmUmUmCmAmCmCmAm | 1 | UmsGfsGmUmGmAfAmGfCmGmAmAfImUfGmCfAmCfAmsGmsGm | 3 |
| AL01051 39 | UmsGmsUmGmCmAmCfUmCfCfGmCmUmUmCmAmCmCmAm | 1 | VpUmsGfsGmUmGmAfAmGfCmGmAmAfImUfGmCfAmCfAmsGmsGm | 4 |
| Notes: "G", "C", "A", "U", "T", and "I" generally represent nucleotides with guanine, cytosine, adenine, uracil, thymine, and hypoxanthine as bases, respectively. Modifications: d represents a deoxynucleotide; m represents 2'-methoxy; f represents 2'-deoxy-2'-fluoro; s represents phosphorothioate; and Vp represents vinylphosphonic acid (a corresponding modification occurs at the 5' terminus of AS). In the sequence listings of the present disclosure, in accordance with the rules of the ST.26 sequence listing rules, "U" is replaced with "T" in the "residues" field for all sequences, "dT" is replaced with "n", and "I" is replaced with "n". In the modified sequences, other modifications, except for Vp, represent modifications to the preceding nucleotide. | | | | |

Table 2. Modified oligonucleotides

| Modified sequence No. | Sense 5'-3' | Sequence ID No. | Anti-sense 5'-3' | Sequence ID No. |
|---|---|---|---|---|
| AL010 5138 | UmsGmsUmGmCmAmCfUmCfCfGmCmUmUmCmAmCmCmAm | 1 | UmsGfsGmUmGmAfAmGfCmGmAmAfImUfGmCfAmCfAmsGmsGm | 3 |
| AL010 5139 | UmsGmsUmGmCmAmCfUmCfCfGmCmUmUmCmAmCmCmAm | 1 | VpUmsGfsGmUmGmAfAmGfCmGmAmAfImUfGmCfAmCfAmsGmsGm | 4 |
| AL010 5140 | UmsGmsUmGmCmAmCfUmCfCfGmCmUmUmCmAmCmCmAm | 1 | Ums(Ggn)GmUmGmAfAmGfCmGmAmAfImUfGmCfAmCfAmsGmsGm | 5 |

(continued)

| Modifi ed sequen ce No. | Sense 5'-3' | Seq uen ce ID No. | Anti-sense 5'-3' | Seq uen ce ID No. |
|---|---|---|---|---|
| AL010 5141 | UmsGmsUmGmCmAmCfUmCfCfGmCmUmUmCmAmCmCmAm | 1 | UmsGfs(Ggn)UmGmAfAmGfCmGmAmAfImUfGmCfAmCfAmsGmsGm | 6 |
| AL010 5142 | UmsGmsUmGmCmAmCfUmCfCfGmCmUmUmCmAmCmCmAm | 1 | UmsGfsGm(Ugn)GmAfAmGfCmGmAmAfImUfGmCfAmCfAmsGmsGm | 7 |
| AL010 5143 | UmsGmsUmGmCmAmCfUmCfCfGmCmUmUmCmAmCmCmAm | 1 | UmsGfsGmUm(Ggn)AfAmGfCmGmAmAfImUfGmCfAmCfAmsGmsGm | 8 |
| AL010 5144 | UmsGmsUmGmCmAmCfUmCfCfGmCmUmUmCmAmCmCmAm | 1 | UmsGfsGmUmGm(Agn)AmGfCmGmAmAfImUfGmCfAmCfAmsGmsGm | 9 |
| AL010 5145 | UmsGmsUmGmCmAmCfUmCfCfGmCmUmUmCmAmCmCmAm | 1 | UmsGfsGmUmGmAf(Agn)GfCmGmAmAfImUfGmCfAmCfAmsGmsGm | 10 |
| AL010 5146 | UmsGmsUmGmCmAmCfUmCfCfGmCmUmUmCmAmCmCmAm | 1 | UmsGfsGmUmGmAfAm(Ggn)CmGmAmAfImUfGmCfAmCfAmsGmsGm | 11 |
| AL010 5147 | UmsGmsUmGmCmAmCfUmCfCfGmCmUmUmCmAmCmCmAm | 1 | UmsG(moe)GmUmGmAfAmGfCmGmAmAfImUfGmCfAmCfAmsGmsGm | 12 |
| AL010 5148 | UmsGmsUmGmCmAmCfUmCfCfGmCmUmUmCmAmCmCmAm | 1 | UmsGfsG(moe)UmGmAfAmGfCmGmAmAfImUfGmCfAmCfAmsGmsGm | 13 |
| AL010 5149 | UmsGmsUmGmCmAmCfUmCfCfGmCmUmUmCmAmCmCmAm | 1 | UmsGfsGmU(moe)GmAfAmGfCmGmAmAfImUfGmCfAmCfAmsGmsGm | 14 |
| AL010 5150 | UmsGmsUmGmCmAmCfUmCfCfGmCmUmUmCmAmCmCmAm | 1 | UmsGfsGmUmG(moe)AfAmGfCmGmAmAfImUfGmCfAmCfAmsGmsGm | 15 |
| AL010 5151 | UmsGmsUmGmCmAmCfUmCfCfGmCmUmUmCmAmCmCmAm | 1 | UmsGfsGmUmGmA(moe)AmGfCmGmAmAfImUfGmCfAmCfAmsGmsGm | 16 |

(continued)

| Modifi ed sequen ce No. | Sense 5'-3' | Seq uen ce ID No. | Anti-sense 5'-3' | Seq uen ce ID No. |
|---|---|---|---|---|
| AL010 5152 | UmsGmsUmGmCmAmCfUmCfCfGmCmUmUmCmAmCmCmAm | 1 | UmsGfsGmUmGmAfA(moe)GfCmGmAmAfImUfGmCfAmCfAmsGmsGm | 17 |
| AL010 5153 | UmsGmsUmGmCmAmCfUmCfCfGmCmUmUmCmAmCmCmAm | 1 | UmsGfsGmUmGmAfAmG(moe)CmGmAmAfImUfGmCfAmCfAmsGmsGm | 18 |
| AL010 5157 | UmsGmsUmGmCmAmCfUmCfCfGmCmUmUmCmAmCmCmAm | 1 | UmsGfsGm(GNF-BX)GmAfAmGfCmGmAmAfImUfGmCfAmCfAmsGmsGm | 19 |
| AL010 5158 | UmsGmsUmGmCmAmCfUmCfCfGmCmUmUmCmAmCmCmAm | 1 | UmsGfsGmUm(GNF-BX)AfAmGfCmGmAmAfImUfGmCfAmCfAmsGmsGm | 20 |
| AL010 5159 | UmsGmsUmGmCmAmCfUmCfCfGmCmUmUmCmAmCmCmAm | 1 | UmsGfsGmUmGm(GNF-BX)AmGfCmGmAmAfImUfGmCfAmCfAmsGmsGm | 21 |
| AL010 5160 | UmsGmsUmGmCmAmCfUmCfCfGmCmUmUmCmAmCmCmAm | 1 | UmsGfsGmUmGmAf(GNF-BX)GfCmGmAmAfImUfGmCfAmCfAmsGmsGm | 22 |
| AL010 5161 | UmsGmsUmGmCmAmCfUmCfCfGmCmUmUmCmAmCmCmAm | 1 | UmsGfsGm(U-2'-5')GmAfAmGfCmGmAmAfImUfGmCfAmCfAmsGmsGm | 23 |
| AL010 5162 | UmsGmsUmGmCmAmCfUmCfCfGmCmUmUmCmAmCmCmAm | 1 | UmsGfsGmUm(G-2'-5')AfAmGfCmGmAmAfImUfGmCfAmCfAmsGmsGm | 24 |
| AL010 5163 | UmsGmsUmGmCmAmCfUmCfCfGmCmUmUmCmAmCmCmAm | 1 | UmsGfsGmUmGm(A-2'-5')AmGfCmGmAmAfImUfGmCfAmCfAmsGmsGm | 25 |
| AL010 5164 | UmsGmsUmGmCmAmCfUmCfCfGmCmUmUmCmAmCmCmAm | 1 | UmsGfsGmUmGmAf(A-2'-5')GfCmGmAmAfImUfGmCfAmCfAmsGmsGm | 26 |
| AL010 5165 | UmsGmsUmGmCmAmCfUmCfCfGmCmUmUmCmAmCmCmAm | 1 | APU001sGfsGmUmGmAfAmGfCmGmAmAfImUfGmCfAmCfAmsGmsGm | 27 |

(continued)

| Modifi ed sequen ce No. | Sense 5'-3' | Seq uen ce ID No. | Anti-sense 5'-3' | Seq uen ce ID No. |
|---|---|---|---|---|
| AL010 5166 | UmsGmsUmGmCmAmCfUmCfCfGmCmUmUmCmAmCmCmAm | 1 | APU001sGfsGmUm(Ggn)AfAmGfCmGmAmAfImUfGmCfAmCfAmsGmsGm | 28 |
| AL010 5167 | UmsGmsUmGmCmAmCfUmCfCfGmCmUmUmCmAmCmCmAm | 1 | APU001sGfsGmUmGmAf(Agn)GfCmGmAmAfImUfGmCfAmCfAmsGmsGm | 29 |
| AL010 5168 | UmsGmsUmGmCmAmCfUmCfCfGmCmUmUmCmAmCmCmAm | 1 | APU001sGfsGmUm(GNF-BX)AfAmGfCmGmAmAfImUfGmCfAmCfAmsGmsGm | 30 |
| AL010 5169 | UmsGmsUmGmCmAmCfUmCfCfGmCmUmUmCmAmCmCmAm | 1 | APU001sGfsGmUmGmAf(GNF-BX)GfCmGmAmAfImUfGmCfAmCfAmsGmsGm | 31 |
| AL010 5170 | UmsGmsUmGmCmAmCfUmCfCfGmCmUmUmCmAmCmCmAm | 1 | APU001sGfsGmUmGmAf(A-2'-5')GfCmGmAmAfImUfGmCfAmCfAmsGmsGm | 32 |
| AL010 5171 | UmsGmsUmGmCmAmCfUfCfCmGmCmUmUmCmAmCmCmAm | 2 | VpUmsGfsGmUmGmAfAmGmCmGmAmAmImUfGmCfAmCmAmsGmsGm | 33 |
| AL010 5172 | UmsGmsUmGmCmAmCfUmCfCfGmCmUmUmCmAmCmCmAm | 1 | VpUmsGfsGmUm(Ggn)AfAmGfCmGmAmAfImUfGmCfAmCfAmsGmsGm | 34 |
| AL010 5173 | UmsGmsUmGmCmAmCfUmCfCfGmCmUmUmCmAmCmCmAm | 1 | VpUmsGfsGmUmGmAf(A-2'-5')GfCmGmAmAfImUfGmCfAmCfAmsGmsGm | 35 |
| Notes: "G", "C", "A", "U", "T", and "I" generally represent nucleotides with guanine, cytosine, adenine, uracil, thymine, and hypoxanthine as bases, respectively. Modifications: d represents a deoxynucleotide; m represents 2'-methoxy; f represents 2'-deoxy-2'-fluoro; Moe represents 2'-O-methoxyethyl; s represents a 3'-phosphorothioate group; and Vp represents 5'-vinylphosphonic acid (a corresponding modification occurs at the 5' terminus of AS). | | | | |

A-2'-5' represents adenosine-2' phosphate. U-2'-5' represents uridine-2' phosphate. G-2'-5' represents guanosine-2' phosphate. Ggn represents guanosine-glycol nucleic acid. Agn represents adenosine-glycol nucleic acid. Ugn represents uridine-glycol nucleic acid. GNF-BX represents N-(2,3-dihydroxypropyl)-3,5-bis(trifluoromethyl)benzamide. APU001 represents 5'-vinylphosphonate-2'-N-acetyl-uridine. With a sequence 3 as a corresponding reference, sequences 4 to 35 are obtained through nucleic acid analog substitutions at corresponding positions.

Tables 3 and 4 list the designed siRNA sequences with carriers.

Table 3. siRNA sequences with carriers

| Car rier sequ ence No. | SS 5'-3' | Sequence ID No./corre sponding modified sequence No. | AS 5'-3' | Seq uen ce ID No. |
|---|---|---|---|---|
| AL0 1070 39 | GmsAmsCmCmGmUmGfUmGfCfAmCmUmUmCmGmCmUmAm_L96 | 52/45 | UmsAfsGmCmGmAfAmGfUmGmCmAfCmAfCmGfImUfCmsCmsGm | 50 |
| AL0 1070 43 | GmsAmsCmCmGmUmGfUmGfCfAmCmUmUmCmGmCmUmAm_L96 | 52/45 | VpUmAfsGmCmGmAfAmGfUmGmCmAfCmAfCmGfImUfCmsCmsGm | 51 |
| AL0 1070 44 | UmsGmsUmGmCmAmCfUmCfCfGmCmUmUmCmAmCmCmAm_L96 | 36/1 | UmsGfsGmUmGmAfAmGfCmGmAmAfImUfGmCfAmCfAmsGmsGm | 3 |
| AL0 1070 45 | UmsGmsUmGmCmAmCfUmCfCfGmCmUmUmCmAmCmCmAm_L96 | 36/1 | VpUmsGfsGmUmGmAfAmGfCmGmAmAfImUfGmCfAmCfAmsGmsGm | 4 |

Table 4. siRNA sequences with carriers

| Carrier sequen ce No. | SS 5'-3' | Seque nce ID No./co rrespo nding modifi ed sequen ce No. | AS 5'-3' | Seque nce ID No. |
|---|---|---|---|---|
| AL010 7044 | UmsGmsUmGmCmAmCfUmCfCfGmCmUmUmCmAmCmCmAm_L96 | 36/1 | UmsGfsGmUmGmAfAmGfCmGmAmAfImUfGmCfAmCfAmsGmsGm | 3 |
| AL010 7045 | UmsGmsUmGmCmAmCfUmCfCfGmCmUmUmCmAmCmCmAm_L96 | 36/1 | VpUmsGfsGmUmGmAfAmGfCmGmAmAfImUfGmCfAmCfAmsGmsGm | 4 |
| AL010 7048 | UmsGmsUmGmCmAmCfUmCfCfGmCmUmUmCmAmCmCmAm_L96 | 36/1 | APU001sGfsGmUmGmAfAmGfCmGmAmAfImUfGmCfAmCfAmsGmsGm | 27 |
| AL010 7049 | UmsGmsUmGmCmAmCfUmCfCfGmCmUmUmCmAmCmCmAm_L96 | 36/1 | APU001sGfsGmUm(Ggn)AfAmGfCmGmAmAfImUfGmCfAmCfAmsGmsGm | 28 |

(continued)

| Carrier sequen ce No. | SS 5'-3' | Seque nce ID No./co rrespo nding modifi ed sequen ce No. | AS 5'-3' | Seque nce ID No. |
|---|---|---|---|---|
| AL010 7050 | UmsGmsUmGmCmAmCf UmCfCfGmCmUmUmCm AmCmCmAm L96 | 36/1 | APU001sGfsGmUmGmAf (Agn)GfCmGmAmAfImUf GmCfAmCfAmsGmsGm | 29 |
| AL010 7051 | UmsGmsUmGmCmAmCf UmCfCfGmCmUmUmCm AmCmCmAm L96 | 36/1 | APU001sGfsGmUm(GNF-BX)AfAmGfCmGmAmAf ImUfGmCfAmCfAmsGms Gm | 30 |
| AL010 7052 | UmsGmsUmGmCmAmCf UmCfCfGmCmUmUmCm AmCmCmAm L96 | 36/1 | APU001sGfsGmUmGmAf (GNF-BX)GfCmGmAmAfImUf GmCfAmCfAmsGmsGm | 31 |
| AL010 7053 | UmsGmsUmGmCmAmCf UmCfCfGmCmUmUmCm AmCmCmAm L96 | 36/1 | APU001sGfsGmUmGmAf (A-2'-5')GfCmGmAmAfImUfG mCfAmCfAmsGmsGm | 32 |
| AL010 7054 | CmsCmsUmUmGmAmGf GfCfAmUmAmCmUmUm CmAmAmAm_L96 | 37 | VpUmsUfsUmGmAmAfG mUmAmUmGmCmCmUf CmAfAmGmGmsUmsUm | 39 |
| AL010 7055 | UmsGmsUmGmCmAmCf UfCfCmGmCmUmUmCm AmCmCmAm_L96 | 38/2 | VpUmsGfsGmUmGmAfA mGmCmGmAmAmImUfG mCfAmCmAmsGmsGm | 33 |
| AL010 7057 | UmsGmsUmGmCmAmCf UmCfCfGmCmUmUmCm AmCmCmAm_L96 | 36/1 | VpUmsGfsGmUm(Ggn)Af AmGfCmGmAmAfImUfG mCfAmCfAmsGmsGm | 34 |
| AL010 7058 | UmsGmsUmGmCmAmCf UmCfCfGmCmUmUmCm AmCmCmAm L96 | 36/1 | VpUmsGfsGmUmGmAf( A-2'-5')GfCmGmAmAfImUfG mCfAmCfAmsGmsGm | 35 |
| Notes: "G", "C", "A", "U", "T", and "I" generally represent nucleotides with guanine, cytosine, adenine, uracil, thymine, and hypoxanthine as bases, respectively. Modifications: d represents a deoxynucleotide; m represents 2'-methoxy; f represents 2'-deoxy-2'-fluoro; Moe represents 2'-O-methoxyethyl; s represents a 3'-phosphorothioate group; and Vp represents 5'-vinylphosphonic acid (a corresponding modification occurs at the 5' terminus of AS). | | | | |

A-2'-5' represents adenosine-2' phosphate. U-2'-5' represents uridine-2' phosphate. G-2'-5' represents guanosine-2' phosphate. Ggn represents guanosine-glycol nucleic acid. Agn represents adenosine-glycol nucleic acid. Ugn represents uridine-glycol nucleic acid. GNF-BX represents N-(2,3-dihydroxypropyl)-3,5-bis(trifluoromethyl)benzamide. APU001 represents 5'-vinylphosphonate-2'-N-acetyl-uridine. With a sequence 3 as a corresponding reference, sequences 4 to 35 are obtained through nucleic acid analog substitutions at corresponding positions.

L96 represents a carrier. Optionally, a ligand conjugated to the oligonucleotide has the following structural formula:

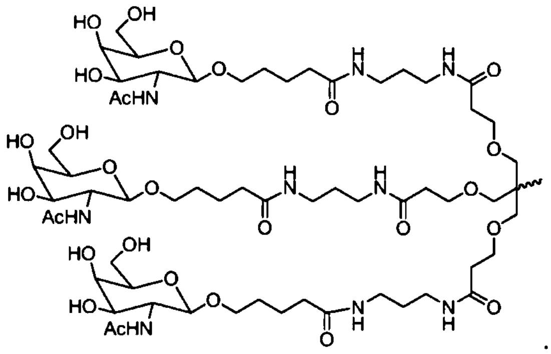

.

Further, the ligand is conjugated to the 3' terminus of the SS of the oligonucleotide through a linker to produce the following conjugate:

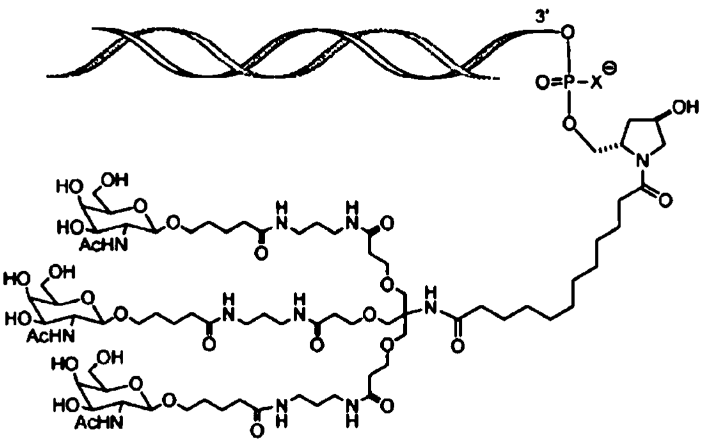

,

wherein X is selected from S or O.

**Example 2: Preparation of siRNA**

Abbreviations for nucleotide monomers are used in nucleic acid sequence listings. It should be understood that these monomers, when present in an oligonucleotide, are linked to each other through 5'-3' phosphodiester linkages, unless otherwise indicated. L96 is prepared with reference to the method described in patent CN104717982B.

A preparation process of an oligonucleotide is specifically as follows:

**(1) Preparation of siRNA**

A siRNA sequence was obtained by separately synthesizing SS and AS on a solid support carrier and then subjecting the synthesized SS and AS to deprotection, cleavage, purification, annealing, purification, and lyophilization.

Solid-phase synthesis (FIG. 1): The SS and the AS were separately synthesized on the solid support carrier using an automated oligonucleotide synthesizer based on phosphoramidite chemistry. The synthesizer was, for example, AKTA Oligopilot (Cytiva) or Dr. Oligo 192XLc (Biolytic Lab Performance Inc.). The solid-phase synthesis proceeded from the 3' terminus of a sequence, and monomers were coupled sequentially to the sequence. The coupling of each phosphoramidite monomer comprised the following four chemical steps: 1) deblocking or deprotection (removal of a hydroxyl protecting group); 2) coupling; 3) oxidation; and 4) capping. The phosphoramidite monomers, reagents, and purification

consumables used were all commercially available reagents and consumables, such as various phosphoramidite monomers (such as 5'-O-(4,4'-dimethoxytrityl)-2'-O-methyl-uridine-3'-CE-phosphoramidite) purchased from Shanghai Hongene Biotech Corporation and reaction reagents (such as 40 wt% methylamine aqueous solution and 28 wt% ammonium hydroxide aqueous solution) purchased from Sigma-Aldrich LLC. The siRNA synthesis and purification methods used herein were described in, for example, US20130178612A1 and US2015100197A1. The synthesis methods for sequences comprising VPUm and APU structures were described in, for example, J. Med. Chem. 2018, 61, 734-744.

**(2) Preparation of double-stranded siRNA reagent**

A synthesis process of SS was as follows:
An oligonucleotide was synthesized by the solid-phase phosphoramidite method. The reaction was carried out in a stainless steel synthesis column using a computer-controlled synthesizer. For the synthesis of the SS, with either a solid support carrier loaded with a targeting ligand (such as L96) or a solid support carrier alone as a starting point, different raw materials, reagents, and solvents were injected in the order from the 3' terminus to the 5' terminus of the sequence through different channels controlled by a solid-phase synthesizer, and phosphoramidite nucleoside monomers were linked one by one. The reaction process involved four-step cycles each including 4,4'-dimethoxytrityl (DMT) protecting group removal, condensation, oxidation or sulfurization, and capping. In each cycle, one nucleotide unit was added. Finally, an oligonucleotide sequence of 19 or 21 nucleotide units was obtained. After the synthesis was completed, a protecting group (2-cyanoethyl) was removed on a solid-phase synthesis column. A synthesized sequence was then cleaved from the solid support carrier through ammonolysis. The cleaved sequence was filtered. The filter cake was washed with ethanol. The filtrate and the washing solution were collected and concentrated to produce a crude SS product. The crude SS product was purified by chromatography (SOURCE 15Q) and lyophilized to produce a target product, which was the SS.

A synthesis process of AS was as follows:
The synthesis of AS was similar to the synthesis of the SS. Different raw materials, reagents, and solvents were injected in the order from the 3' terminus to the 5' terminus of the sequence through different channels controlled by a solid-phase synthesizer, and phosphoramidite nucleoside monomers were linked one by one. The reaction process involved four-step cycles each including DMT protecting group removal, condensation, oxidation or sulfurization, and capping. In each cycle, one nucleotide unit was added. Finally, an oligonucleotide sequence of 21 or 23 nucleotide units was obtained. After the synthesis was completed, a protecting group (2-cyanoethyl) was removed on a solid-phase synthesis column. A synthesized sequence was then cleaved from the solid support carrier through ammonolysis. The cleaved sequence was filtered. The filter cake was washed with ethanol. The filtrate and the washing solution were collected and concentrated to produce a crude AS product. The crude AS product was purified by chromatography (SOURCE 15Q), ultrafiltered, and lyophilized to produce a target product, which was the AS.

A preparation process of the double-stranded siRNA product was as follows:
The AS and the SS were dissolved in water for injection separately, mixed at a specified ratio (1.01:1.0 to 1.2:1.0), incubated at 30°C to 50°C for 30 min to 90 min, then cooled to room temperature, and lyophilized to produce the double-stranded siRNA product.

Following the same method, the double-stranded siRNA conjugates shown in Tables 1 and 2 as described above were prepared.

**Example 3: Evaluation of *in vitro* anti-HBV activities of modified sequences using HepG2.2.15 cells**

(1) Culture of HepG2.2.15 cells:

HepG2.2.15 cells are HepG2 cells transfected with a recombinant HBV-DNA plasmid, which can stably produce HBV. Thus, the HepG2.2.15 cells are a common *in vitro* model for HBV drug screening. The HepG2.2.15 cells were provided by WuXi AppTec (Shanghai) Co., Ltd. and cultured in a 37°C, 5% $CO_2$ incubator using a DMEM basal medium (Gibco-11995-065) supplemented with 10% of fetal bovine serum (FBS) (ExCell Bio-FSP500), InvitroGRO CP Medium (BioreclamationIVT-S03316), and 1% of penicillin-streptomycin (Hyclone-SV30010).

(2) Plating and transfection

On day 0, a test compound was first serially diluted with Dulbecco's Phosphate-Buffered Saline (DPBS) (KCl, 0.2 g/L; $KH_2PO_4$ 0.2 g/L; NaCl, 8 g/L; $Na_2HPO_4 \cdot 7H_2O$, 2.16 g/L; pH=7.0) to final concentrations of 1 nM and 0.1 nM. The HepG2.2.15 cells growing in a culture flask were taken, washed with DPBS, and then digested with trypsin.

The collected cells were counted, adjusted to an appropriate density, and then inoculated into a 96-well plate at a density of $1.5 \times 10^4$ cells/well. While the cells were inoculated, the test compound was transfected into the cells using

Lipofectamine RNAiMax (Invitrogene, 13378-150). Three replicate wells were set for each concentration of each test compound.

(3) Sample collection and detection

72 h after the transfection of the test compound, a cell culture supernatant was collected, and an expression level of HBsAg in the cell culture supernatant was detected by enzyme-linked immunosorbent assay (ELISA). The cell culture supernatant was diluted with DPBS, and then the HBsAg content was detected using an HBsAg ELISA kit (Catalog No.: Autobio-CL0310). The method was briefly described as follows: a standard, a sample, and a control were each taken in 50 μL and added to an assay plate, 50 μL of enzyme conjugate was then added to each well, and the above mixtures were incubated at 37°C for 60 min; the assay plate was washed with a washing solution and then suck-dried; 50 μL of premixed luminescent substrate was added, and the mixtures were incubated at room temperature in the dark for 10 min; and finally, a luminescence value was measured using a microplate reader.

(4) Data analysis

HBsAg inhibition rate (%) = (1 - HBsAg value of sample/HBsAg value of DPBS control) × 100%.

Calculation results for the *in vitro* inhibitory activity on the HepG2.2.15 cells were shown in Table 5.

Table 5. *In vitro* inhibitory activities on HepG2.2.15 cells (n = 3)

| Compound No. | HBsAg Inhibition (%) | |
|---|---|---|
| | 0.1 nM | 0.01 nM |
| | MEAN±standard deviation (SD) | MEAN±SD |
| AL0105125 | 64.37±3.96 | 30.06±8.27 |
| AL0105127 | 72.06±0.47 | 45.48±1.41 |
| AL0105128 | 54.25±1.40 | 31.82±9.12 |
| AL0105131 | 72.56±2.21 | 42.87±7.84 |
| AL0105132 | 71.07±3.41 | 46.51±2.89 |
| AL0105133 | 71.57±0.70 | 51.92±1.07 |
| AL0105134 | 43.70±5.41 | 25.45±4.40 |
| AL0105137 | 83.58±2.06 | 78.53±5.67 |
| AL0105138 | 73.76±2.21 | 46.09±2.90 |
| AL0105139 | 83.90±1.19 | 64.72±2.29 |

According to the above results, among the drugs with different modifications, AL0105125, AL0105128, and AL0105134 exhibited relatively low inhibitory efficiency against HBsAg, and all other siRNA drugs exhibited HBsAg inhibition efficiency of 70% or more at a concentration of 0.1 nM and HBsAg inhibition efficiency of 40% or more at a concentration of 0.01 nM. AL0105133 and AL0105138 showed particularly prominent inhibitory effects. AL0105137 and AL0105139, which were obtained by introducing a Vp modification at 5' termini of ASs of AL0105133 and AL0105138, showed further enhanced inhibitory effects.

**Example 4: Evaluation of *in vitro* anti-HBV activities of modified sequences using HepG2.2.15 cells**

(1) Culture of HepG2.2.15 cells:

HepG2.2.15 cells are HepG2 cells transfected with a recombinant HBV-DNA plasmid, which can stably produce HBV. Thus, the HepG2.2.15 cells are a common *in vitro* model for HBV drug screening. The HepG2.2.15 cells were provided by WuXi AppTec (Shanghai) Co., Ltd. and cultured in a 37°C, 5% $CO_2$ incubator using a DMEM basal medium (Gibco-11995-065) supplemented with 10% of FBS (ExCell Bio-FSP500), InvitroGRO CP Medium (BioreclamationIVT-S03316), and 1% of penicillin-streptomycin (Hyclone-SV30010).

(2) Plating and transfection

On day 0, a test compound was first serially diluted with DPBS (KCl, 0.2 g/L; $KH_2PO_4$, 0.2 g/L; NaCl, 8 g/L; $Na_2HPO_4 \cdot 7H_2O$, 2.16 g/L; pH=7.0) to final concentrations of 1 nM and 0.1 nM. The HepG2.2.15 cells growing in a culture flask were taken, washed with DPBS, and then digested with trypsin.

The collected cells were counted, adjusted to an appropriate density, and then inoculated into a 96-well plate at a density of $1.5 \times 10^4$ cells/well. While the cells were inoculated, the test compound was transfected into the cells using Lipofectamine RNAiMax (Invitrogene, 13378-150). Three replicate wells were set for each concentration of each test compound.

(3) Sample collection and detection

72 h after the transfection of the test compound, a cell culture supernatant was collected, and an expression level of HBsAg in the cell culture supernatant was detected by ELISA. The cell culture supernatant was diluted with DPBS, and then the HBsAg content was detected using an HBsAg ELISA kit (Catalog No.: Autobio-CL0310). The method was briefly described as follows: a standard, a sample, and a control were each taken in 50 μL and added to an assay plate; 50 μL of enzyme conjugate was then added to each well, and the above mixture was incubated at 37°C for 60 min; the assay plate was washed with a washing solution and then suck-dried; 50 μL of premixed luminescent substrate was added, and the mixture was incubated at room temperature in the dark for 10 min; and finally, a luminescence value was measured using a microplate reader.

(4) Data analysis

HBsAg inhibition rate (%) = (1 - HBsAg value of sample/HBsAg value of DPBS control) $\times$ 100%.

Calculation results for the *in vitro* inhibitory activity on the HepG2.2.15 cells were shown in Table 6.

Table 6. *In vitro* inhibitory activities on HepG2.2.15 cells (n = 3)

| Compound No. | HBsAg Inhibition (%) | |
|---|---|---|
| | 0.1 nM | 0.01 nM |
| | MEAN±SD | MEAN±SD |
| AL0105138 | 76.62±4.73 | 57.67±0.76 |
| AL0105140 | 29.46±5.61 | 29.95±6.83 |
| AL0105141 | 89.42±1.21 | 77.19±1.67 |
| AL0105142 | 68.27±3.14 | 44.64±2.28 |
| AL0105143 | 88.86±1.29 | 81.35±2.06 |
| AL0105144 | 74.26±0.57 | 57.46±1.50 |
| AL0105145 | 75.76±3.52 | 38.20±6.60 |
| AL0105146 | 74.14±2.04 | 47.65±5.30 |
| AL0105147 | 9.57±24.27 | -21.44±5.06 |
| AL0105148 | 58.50±3.94 | 20.49±6.88 |
| AL0105149 | 59.04±2.56 | 20.53±1.28 |
| AL0105150 | 77.55±4.59 | 56.74±2.08 |
| AL0105151 | 56.72±2.20 | 38.97±1.89 |
| AL0105152 | 64.70±4.90 | 43.00±5.85 |

(continued)

| Compound No. | HBsAg Inhibition (%) | |
|---|---|---|
| | 0.1 nM | 0.01 nM |
| | MEAN±SD | MEAN±SD |
| AL0105153 | 78.49±1.67 | 57.16±2.74 |

According to the above results, with the exception of compounds AL0105140, AL0105147, AL0105148, AL0105149, and AL0105151, all other compounds exhibited relatively high HBsAg inhibition levels, with average inhibition rates of 75% or more. Inhibition rates of compounds AL0105141 and AL0105143 were 90% or more.

**Example 5: Evaluation of *in vitro* off-target activities of modified sequences using HepG2.2.15 cells**

**(1) Experimental method**

In this experiment, a psi-CHECK2 (Promega TM) plasmid system was employed to evaluate the intrinsic on-target and off-target effects of guide strand and passenger strand pairs by monitoring a change in an expression level of a target sequence. For each siRNA, two psi-CHECK2 plasmids were prepared: one comprised the complete target sequence (a sequence perfectly complementary to the guide strand or the passenger strand of the corresponding siRNA), and the other comprised a sequence not perfectly matching the target sequence. These sequences were cloned downstream of a coding region of a Renilla luciferase reporter gene. siRNA with RNAi activity or seed-mediated activity targeting any of the above sequences could lead to cleavage and subsequent degradation or translational inhibition of the fusion mRNA. Any of these conditions led to a decline in protein expression.

**(2) Data analysis**

$$\text{Inhibition efficiency (\%)} = 1 - (\text{ratio reporter + siRNA/ratio reporter only}) * 100\%.$$

$IC_{50}$ was calculated using the "[inhibitor] vs. normalized response - variable slope" function in Graphpad Prism 7. This function employed the following equation:

$$(Y = 100/(1 + (X\text{\^{}HillSlope})/(IC_{50}\text{\^{}HillSlope}))).$$

Experimental results were shown in Table 7.

Table 7. *In vitro* inhibitory activities on HepG2.2.15 cells (n = 3)

| Compound No. | On-target $IC_{50}$ (nM) | Off-target $IC_{50}$ (nM) |
|---|---|---|
| AL0105138 | 0.01808 | 0.1481 |
| AL0105157 | 0.00384 | > 40 |
| AL0105158 | 0.001813 | > 40 |
| AL0105159 | 0.01209 | 0.7491 |
| AL0105160 | 0.002436 | 0.2208 |
| AL0105161 | 0.113 | 0.4891 |
| AL0105162 | 0.0194 | 0.1512 |
| AL0105163 | 0.0411 | 0.9129 |
| AL0105164 | 0.001835 | 0.542 |

According to the above results, regarding on-target activity, except for AL0105161 which exhibited significantly reduced inhibitory efficiency, all other siRNA drugs demonstrated equal or better inhibitory efficiency to or than

AL0105138. In particular, AL0105157, AL0105158, AL0105159, AL0105160, and AL0105164 had significantly higher inhibitory efficiency than AL0105138. Regarding off-target activity, all compounds produced after different optimizations of AL0105138 could significantly improve the OTE of AL0105138.

## Example 6: Evaluation of *in vivo* anti-HBV activities of compounds at different concentrations using HBV transgenic mice

In this experiment, SPF-grade male C57B/6N-Tg (1.28HBV)/Vst mice aged 6 weeks to 8 weeks (Beijing Vitalstar Biotechnology Co., Ltd., Animal Production License No. SCXK (Beijing) 2019-0002) were employed. Mice were raised separately with a single animal in each cage. The use and testing of animal feed, bedding, and drinking water were performed in accordance with the specifications of GB14925-2010 *"Laboratory animal - Requirements of environment and housing facilities"*.

After one week of acclimatization, the experiment was initiated. Based on quantitative detection results of serum HBsAg (primary) and HBV DNA (secondary), the mice were randomly divided into 9 groups with 5 mice per group. The day of administration was recorded as D1. On the day of administration, blood was collected prior to administration and tested for HBsAg, HBV DNA, and HBeAg.

All animals were administered on D1 through a single subcutaneous injection. AL0107039 and AL0107044 were each administered at doses of 1 mg/kg, 3 mg/kg, and 9 mg/kg, and AL0107043 and AL0107045 were each administered at a dose of 3 mg/kg, with an administration volume of 5 mL/kg. Normal saline was injected at an equivalent dose to create a control group. At 15 min to 1 h after the last animal in a group was administered, the animals in this group were observed.

On day 8, day 15, day 22, day 29, day 35, and day 42 after administration, 100 μL of blood was collected from the orbit, and serum was isolated through centrifugation. 10 μL of serum was taken, diluted 50-fold with PBS, and submitted for testing. Diluted serum samples were tested for HBsAg, HBeAg, and HBV DNA by Beijing Dian Medical Inspection Laboratory Co., Ltd.

Experimental data was subjected to statistical analysis with statistical analysis software Graphpad prism. The residual inhibition rates of HBV DNA, HBsAg, and HBeAg were all calculated with an average of two measurements before administration as a baseline value:

residual inhibition rate = (value measured at different time points/baseline value before administration) * 100%.

Experimental results are shown in Table 8 and FIG. 2.

Table 8. Residual rates of serum HBsAg, HBeAg, and HBV DNA in HBV transgenic mice after a single subcutaneous administration at different concentrations

| Ti me | Index | 1 mg/kg | | 3 mg/kg | | | | 9 mg/kg | |
|---|---|---|---|---|---|---|---|---|---|
| | | AL010 7039 | AL010 7044 | AL010 7039 | AL010 7044 | AL010 7043 | AL010 7045 | AL010 7039 | AL010 7044 |
| D8 | HBsAg | 10.74% | 16.95% | 2.03% | 3.32% | 0.76% | 0.60% | 0.40% | 1.44% |
| | HBV DNA | 43.16% | 55.92% | 14.20% | 22.30% | 4.32% | 6.75% | 2.53% | 12.27% |
| | HBeAg | 54.67% | 62.61% | 34.39% | 39.69% | 26.99% | 26.39% | 26.29% | 26.70% |
| D1 5 | HBsAg | 15.48% | 23.31% | 2.88% | 2.32% | 0.85% | 0.21% | 0.34% | 0.45% |
| | HBV DNA | 53.91% | 80.61% | 19.62% | 15.62% | 5.09% | 3.78% | 1.94% | 3.40% |
| | HBeAg | 51.18% | 60.56% | 30.79% | 32.29% | 24.43% | 22.85% | 21.09% | 24.77% |
| D2 2 | HBsAg | 34.14% | 23.42% | 6.19% | 2.75% | 2.72% | 0.23% | 0.95% | 0.67% |
| | HBV DNA | 120.92 % | 99.45% | 56.21% | 23.89% | 9.99% | 6.88% | 10.12% | 9.85% |
| | HBeAg | 64.40% | 68.18% | 41.77% | 38.19% | 30.73% | 28.24% | 29.13% | 30.22% |
| D2 9 | HBsAg | 35.58% | 25.48% | 11.47% | 4.34% | 2.50% | 0.29% | 1.58% | 1.57% |
| | HBV DNA | 115.68 % | 90.51% | 67.66% | 38.62% | 7.81% | 5.35% | 15.44% | 8.24% |
| | HBeAg | 81.57% | 74.86% | 50.53% | 40.95% | 31.70% | 29.54% | 37.57% | 31.88% |
| D3 5 | HBsAg | 38.44% | 38.76% | 15.85% | 6.97% | 7.83% | 0.49% | 4.03% | 4.89% |
| | HBV DNA | 99.39% | 119.47 % | 64.41% | 45.12% | 24.85% | 7.81% | 20.39% | 17.57% |
| | HBeAg | 83.92% | 87.31% | 52.31% | 46.86% | 28.55% | 20.80% | 38.16% | 41.86% |
| D4 2 | HBsAg | 51.59% | 41.92% | 25.02% | 12.32% | 9.87% | 0.74% | 8.64% | 7.54% |
| | HBV DNA | 88.40% | 110.52 % | 70.88% | 44.24% | 31.17% | 11.06% | 52.99% | 25.28% |
| | HBeAg | 87.01% | 78.33% | 63.19% | 51.26% | 46.40% | 30.51% | 46.95% | 39.45% |

According to the test results shown in FIG. 2 and Table 8, compounds AL0107039 and AL0107044 both exhibited significant inhibitory effects on HBsAg, HBV DNA, and HBeAg in HBV transgenic mice in a clearly dose-dependent manner, and could show significant inhibitory efficiency at a minimum dose of 1 mg/kg. AL0107044 had a better inhibitory effect than AL0107039, and AL0107045 had a better inhibitory effect than AL0107043.

**Example 7: Evaluation of *in vivo* anti-HBV activities of compounds using HBV transgenic mice**

In this experiment, SPF-grade male C57B/6N-Tg (1.28HBV)/Vst mice aged 6 weeks to 8 weeks (Beijing Vitalstar Biotechnology Co., Ltd., Animal Production License No. SCXK (Beijing) 2019-0002) were employed. Mice were raised separately with a single animal in each cage. The use and testing of animal feed, bedding, and drinking water were performed in accordance with the specifications of GB14925-2010 *"Laboratory animal - Requirements of environment and housing facilities"*.

After one week of acclimatization, the experiment was initiated. Based on quantitative detection results of serum HBsAg (primary) and HBV DNA (secondary), the mice were randomly divided into 9 groups with 5 mice per group. The day of administration was recorded as D1. On the day of administration, blood was collected prior to administration and tested for HBsAg, HBV DNA, and HBeAg.

All animals were administered on D1 through a single subcutaneous injection, with an administration dose of 3 mg/kg and an administration volume of 5 mL/kg. Normal saline was injected at an equivalent dose to create a control group. At 15 min to 1 h after the last animal in a group was administered, animals in this group were observed.

On day 8, day 15, day 22, and day 29 after administration, 100 μL of blood was collected from the orbit, and serum was isolated through centrifugation. 10 μL of serum was taken, diluted 50-fold with PBS, and submitted for testing. Diluted serum samples were tested for HBsAg, HBeAg, and HBV DNA by Beijing Dian Medical Inspection Laboratory Co., Ltd.

Experimental data was subjected to statistical analysis with the statistical analysis software Graphpad prism. The residual inhibition rates of HBV DNA, HBsAg, and HBeAg were all calculated with an average of two measurements before administration as a baseline value: residual inhibition rate = (value measured at different time points/baseline value before administration) * 100%.

Experimental results are shown in Tables 9 to 11 and FIG. 3.

Table 9. Inhibitory effects on serum HBV DNA in HBV transgenic mice after single subcutaneous administration [$Log_{10}$HBV DNA(IU/mL)] (mean±SD)

| | D1 | D7 | D14 | D21 | D28 | D35 |
|---|---|---|---|---|---|---|
| AL0107045 | 8.16±0.21 | 7.21±0.90 | 6.65±0.79 | 6.58±0.95 | 7.04±0.93 | 6.56±0.90 |
| AL0107048 | 8.09±0.05 | 7.10±0.28 | 6.72±0.63 | 7.10±0.42 | 7.27±0.83 | 7.03±0.82 |
| AL0107049 | 8.14±0.19 | 7.11±0.37 | 7.42±0.42 | 7.66±0.18 | 7.90±0.39 | 7.78±0.36 |
| AL0107050 | 8.26±0.11 | 7.89±0.14 | 7.84±0.14 | 7.97±0.14 | 8.15±0.08 | 8.11±0.08 |
| AL0107051 | 8.08±0.25 | 7.64±0.39 | 7.90±0.51 | 8.14±0.14 | 8.12±0.08 | 8.22±0.05 |
| AL0107052 | 8.16±0.18 | 7.82±0.07 | 7.88±0.06 | 7.99±0.19 | 7.99±0.20 | 7.97±0.27 |
| AL0107053 | 8.12±0.21 | 7.43±0.18 | 7.19±0.34 | 7.45±0.24 | 7.75±0.26 | 7.89±0.35 |
| AL0107054 | 8.19±0.14 | 7.32±0.23 | 7.43±0.28 | 7.48±0.26 | 7.62±0.28 | 7.54±0.61 |
| Normal saline | 8.24±0.20 | 8.31±0.07 | 8.29±0.23 | 8.43±0.13 | 8.28±0.18 | 8.19±0.16 |

Table 10. Inhibitory effects on serum HBeAg in HBV transgenic mice after single subcutaneous administration [$Log_{10}$HBeAg(IU/mL)] (mean±SD)

| | D1 | D7 | D14 | D21 | D28 | D35 |
|---|---|---|---|---|---|---|
| AL0107045 | 1.63±0.03 | 1.00±0.03 | 1.11±0.03 | 0.96±0.02 | 1.03±0.04 | 1.19±0.03 |
| AL0107048 | 1.63±0.05 | 1.01±0.06 | 1.08±0.04 | 1.00±0.07 | 1.12±0.07 | 1.36±0.05 |
| AL0107049 | 1.60±0.02 | 1.07±0.12 | 1.23±0.12 | 1.25±0.09 | 1.43±0.11 | 1.57±0.04 |
| AL0107050 | 1.65±0.04 | 1.17±0.06 | 1.31±0.05 | 1.30±0.04 | 1.46±0.02 | 1.66±0.03 |
| AL0107051 | 1.64±0.04 | 1.30±0.04 | 1.51±0.03 | 1.50±0.03 | 1.68±0.06 | 1.72±0.03 |
| AL0107052 | 1.62±0.02 | 1.19±0.03 | 1.30±0.01 | 1.30±0.04 | 1.45±0.02 | 1.60±0.04 |

(continued)

| | D1 | D7 | D14 | D21 | D28 | D35 |
|---|---|---|---|---|---|---|
| AL0107053 | 1.64±0.02 | 1.01±0.05 | 1.12±0.09 | 1.08±0.05 | 1.24±0.08 | 1.49±0.07 |
| AL0107054 | 1.64±0.04 | 0.98±0.06 | 1.10±0.06 | 1.06±0.04 | 1.13±0.05 | 1.28±0.02 |
| Normal saline | 1.61±0.03 | 1.66±0.02 | 1.65±0.11 | 1.66±0.02 | 1.71±0.08 | 1.78±0.03 |

Table 11. Inhibitory effects on serum HBsAg in HBV transgenic mice after single subcutaneous administration [$Log_{10}$HBsAg(IU/mL)] (mean±SD)

| | D1 | D7 | D14 | D21 | D28 | D35 |
|---|---|---|---|---|---|---|
| AL0107045 | 3.73±0.15 | 1.42±0.66 | 1.06±0.56 | 1.15±0.58 | 1.44±0.74 | 1.49±0.76 |
| AL0107048 | 3.79±0.15 | 1.78±0.32 | 1.40±0.31 | 1.76±0.32 | 2.06±0.46 | 2.39±0.50 |
| AL0107049 | 3.76±0.10 | 1.71±0.52 | 1.88±0.59 | 2.60±0.45 | 3.02±0.63 | 3.20±0.58 |
| AL0107050 | 3.74±0.10 | 2.29±0.26 | 2.46±0.19 | 2.90±0.14 | 3.36±0.12 | 3.55±0.12 |
| AL0107051 | 3.80±0.15 | 2.38±0.30 | 2.79±0.43 | 3.33±0.26 | 3.72±0.16 | 3.73±0.11 |
| AL0107052 | 3.76±0.07 | 2.32±0.18 | 2.36±0.15 | 2.76±0.15 | 3.21±0.15 | 3.23±0.20 |
| AL0107053 | 3.77±0.07 | 1.54±0.30 | 1.42±0.29 | 2.16±0.21 | 2.68±0.31 | 2.98±0.34 |
| AL0107054 | 3.76±0.10 | 1.68±0.27 | 1.62±0.29 | 2.03±0.29 | 2.27±0.32 | 2.22±0.62 |
| Normal saline | 3.76±0.09 | 3.70±0.04 | 3.68±0.07 | 3.79±0.05 | 3.83±0.17 | 3.65±0.19 |

According to the test results shown in Tables 9 to 11 and FIG. 3, all compounds exhibited significant inhibitory effects on HBsAg, HBV DNA, and HBeAg in HBV transgenic mice. Compounds AL0107045 and AL0107048 exhibited the optimal inhibitory effects. Optimized compounds (AL0107049 to AL0107054) had slightly lower inhibitory efficiency than the original compound AL0107048. Among the optimized compounds (AL0107049 to AL0107054), AL0107049 and AL0107053 retained relatively prominent inhibitory efficiency.

**Example 8: Evaluation of *in vivo* anti-HBV activities of different compounds using HBV transgenic mice**

In this experiment, SPF-grade male C57B/6N-Tg (1.28HBV)/Vst mice aged 6 weeks to 8 weeks (Beijing Vitalstar Biotechnology Co., Ltd., Animal Production License No. SCXK (Beijing) 2019-0002) were employed. Mice were raised separately with a single animal in each cage. The use and testing of animal feed, bedding, and drinking water were performed in accordance with the specifications of GB14925-2010 *"Laboratory animal - Requirements of environment and housing facilities"*.

After one week of acclimatization, the experiment was initiated. Based on quantitative detection results of serum HBsAg (primary) and HBV DNA (secondary), the mice were randomly divided into 10 groups with 5 mice per group. The day of administration was recorded as D1. On the day of administration, blood was collected prior to administration and tested for HBsAg, HBV DNA, and HBeAg.

All animals were administered on D1 through a single subcutaneous injection, with an administration dose of 3 mg/kg and an administration volume of 5 mL/kg. Normal saline was injected at an equivalent dose to create a control group. On day 7, day 14, day 21, day 28, and day 35 after administration, 100 μL of blood was collected from the orbit, and serum was isolated through centrifugation. 10 μL of serum was taken, diluted 50-fold with PBS, and submitted for testing. Diluted serum samples were tested for HBsAg, HBeAg, and HBV DNA by Beijing Dian Medical Inspection Laboratory Co., Ltd.

Experimental data was subjected to statistical analysis with the statistical analysis software Graphpad prism.

Experimental results are shown in Tables 12 to 14 and FIG. 4.

Table 12. Inhibitory effects on serum HBV DNA in HBV transgenic mice after single subcutaneous administration [$Log_{10}$HBV DNA(IU/mL)] (mean±SD)

| | D1 | D7 | D14 | D21 | D28 | D35 |
|---|---|---|---|---|---|---|
| AL0107045 | 8.19±0.11 | 7.24±0.09 | 7.09±0.09 | 7.28±0.12 | 7.12±0.09 | 7.27±0.15 |
| AL0107057 | 8.11±0.16 | 6.99±0.06 | 6.95±0.19 | 7.14±0.20 | 7.14±0.20 | 7.42±0.22 |

(continued)

| | D1 | D7 | D14 | D21 | D28 | D35 |
|---|---|---|---|---|---|---|
| AL0107058 | 8.05±0.13 | 6.97±0.23 | 6.60±0.55 | 6.73±0.57 | 6.82±0.38 | 7.10±0.35 |
| Normal saline | 8.12±0.20 | 8.15±0.11 | 8.29±0.04 | 8.25±0.06 | 8.11±0.03 | 8.18±0.07 |

Table 13. Inhibitory effects on serum HBeAg in HBV transgenic mice after single subcutaneous administration [$Log_{10}$HBeAg(IU/mL)] (mean±SD)

| | D1 | D7 | D14 | D21 | D28 | D35 |
|---|---|---|---|---|---|---|
| AL0107045 | 1.64±0.02 | 0.93±0.03 | 0.81±0.04 | 0.89±0.04 | 1.04±0.04 | 1.06±0.04 |
| AL0107057 | 1.66±0.03 | 0.93±0.07 | 0.85±0.06 | 0.94±0.07 | 1.13±0.04 | 1.17±0.10 |
| AL0107058 | 1.66±0.03 | 0.89±0.07 | 0.81±0.06 | 0.84±0.06 | 0.99±0.08 | 1.05±0.05 |
| Normal saline | 1.65±0.05 | 1.61±0.04 | 1.60±0.03 | 1.63±0.04 | 1.85±0.04 | 1.75±0.04 |

Table 14. Inhibitory effects on serum HBsAg in HBV transgenic mice after single subcutaneous administration [$Log_{10}$HBsAg(IU/mL)] (mean±SD)

| | D1 | D7 | D14 | D21 | D28 | D35 |
|---|---|---|---|---|---|---|
| AL0107045 | 3.87±0.07 | 1.93±0.03 | 1.40±0.07 | 1.70±0.04 | 1.94±0.12 | 2.15±0.15 |
| AL0107057 | 3.86±0.11 | 1.71±0.11 | 1.48±0.19 | 1.75±0.21 | 2.09±0.25 | 2.33±0.23 |
| AL0107058 | 3.87±0.08 | 1.63±0.29 | 1.18±0.39 | 1.32±0.48 | 1.57±0.37 | 1.86±0.35 |
| Normal saline | 3.88±0.08 | 3.82±0.12 | 3.84±0.07 | 3.85±0.04 | 3.98±0.06 | 3.91±0.06 |

According to the test results shown in Tables 12 to 14 and FIG. 4, compounds AL0107045, AL0107057, and AL0107058 all exhibited a significant inhibitory effect on HBsAg, HBV DNA, and HBeAg in HBV transgenic mice, and an inhibitory effect of the compound AL0107058 was particularly remarkable. Compared to the original sequence AL0107045, AL0107058 unexpectedly showed an enhanced inhibitory effect on HBsAg, HBV DNA, and HBeAg, which persisted throughout the experimental period.

**Example 9: Evaluation of *in vivo* toxicity of compounds at different concentrations using SD rats**

In this experiment, 86 quarantine-qualified SPF-grade SD rats (half male and half female) were selected and randomly divided into the following groups based on body weights: a vehicle control group (group N, 0.9% sodium chloride injection), an AL0107045S group, an AL7045M group, an AL7045H group, an AL0107057S group, an AL0107057M group, an AL0107057H group, an AL0107058S group, an AL0107058M group, and an AL0107058H group, with 8 rats that were half male and half female in each group. After the grouping, each rat was subcutaneously injected with a test sample/vehicle three times (on day 1, day 15, and day 29 for AL0107045, AL0107057, and AL0107058 studies). The day of the first administration of test samples was designated as day 1 of a study. S, M, and H groups for each test sample were administered at doses of 50.0 mg/kg/administration, 100.0 mg/kg/administration, and 300.0 mg/kg/administration BW (body weight), respectively. The group N was administered with the 0.9% sodium chloride injection. An administration volume for each group was 5.0 mL/kg/administration. The body weight was measured on day 1, day 5, day 7, day 12, day 14, day 19, day 21, day 26, and day 28 of each study. On the day of the last administration, all animals were fasted overnight and then anesthetized by intraperitoneal injection. Blood was collected from the abdominal vein, and serum was isolated and subjected to biochemical analysis. Experimental results are shown in FIG. 5 to FIG. 6.

According to the body weight change results (FIG. 5), notably, animals in all dose groups of AL0107045 underwent a slow body weight gain on day 7 to day 14 of an experiment compared to animals in the normal saline group, and even underwent a negative body weight growth on day 12 to day 14. Such a response was consistent between male and female animals. A body weight gain returned to normal on day 14 to day 28. All dose groups of AL0107057 and AL0107058 showed no significant difference in body weight gain from the normal saline group.

According to GOT results (FIG. 6), apparently, animals in all dose groups of AL0107045 had significantly higher GOT levels than animals in the normal saline group, and all dose groups of AL0107057 and AL0107058 had no significant impact on GOT level.

Based on the body weight change and GOT results, it could be inferred that the compound AL0107045 had a

significant toxic effect on rats, causing a body weight loss during an experiment. Further, according to results of biochemical analysis in serum after the experiment, the GOT level remarkably increased in the AL0107045 groups, indicating that the compound AL0107045 induced a toxic response to the liver. The compounds AL0107057 and AL0107058 were obtained by optimizing AL0107045. According to the test results, the AL0107057 and AL0107058 groups were not much different from the control group in terms of either body weight gain or GOT level. Evidently, the compounds AL0107057 and AL0107058 exhibited improved toxicity profiles compared to AL0107045.

The technical characteristics of the above embodiments can be arbitrarily combined. For brevity of description, not all possible combinations of the technical characteristics of the above embodiments are described. However, these combinations of the technical characteristics should be construed as falling within the scope defined by the specification as long as there is no contradiction among the combinations.

The above embodiments are merely some implementations of the present disclosure, and the description thereof is specific and detailed, but should not be construed as limiting the patent scope of the present disclosure. It should be noted that those of ordinary skill in the art can further make several variations and improvements without departing from the concept of the present disclosure, and these variations and improvements all fall within the scope of protection of the present disclosure. Therefore, the scope of protection of the present disclosure should be subject to the claims.

## Claims

**1.** An oligonucleotide for inhibiting hepatitis B virus (HBV) or a pharmaceutically acceptable salt thereof, wherein the oligonucleotide comprises a sense strand (SS) and an antisense strand (AS), wherein,

the nucleotide sequence of the SS comprises a sequence shown in SEQ ID NO: 1 or a fragment thereof; and
the nucleotide sequence of the AS comprises a sequence shown in SEQ ID NO: 3 or a fragment thereof.

**2.** The oligonucleotide or the pharmaceutically acceptable salt thereof according to claim 1, wherein

preferably, the AS is further subjected to a modification, and the modification is selected from one or more of a 2'-methoxyethyl modification, a 2'-methoxy (m) modification, a 2'-deoxy-2'-fluoro (f) modification, a phosphorothioate group (s) modification, an adenosine-2' phosphate (A-2'-5') modification, a uridine-2' phosphate (U-2'-5') modification, a guanosine-2' phosphate (G-2'-5') modification, a guanosine-glycol nucleic acid (Ggn) modification, an adenosine-glycol nucleic acid (Agn) modification, an N-(2,3-dihydroxypropyl)-3,5-bis(trifluoromethyl)benzamide (GNF-BX) modification, or a 5'-phosphate mimic modification;
preferably, the modification is selected from one or more of a 2'-methoxyethyl modification, an adenosine-2' phosphate (A-2'-5') modification, a uridine-2' phosphate (U-2'-5') modification, a guanosine-2' phosphate (G-2'-5') modification, a guanosine-glycol nucleic acid (Ggn) modification, an adenosine-glycol nucleic acid (Agn) modification, an N-(2,3-dihydroxypropyl)-3,5-bis(trifluoromethyl)benzamide (GNF-BX) modification, or a 5'-phosphate mimic modification;
preferably, the modification is a combination of either a guanosine-glycol nucleic acid (Ggn) modification or an adenosine-2' phosphate (A-2'-5') modification with a 5'-phosphate mimic modification;
preferably, the modification is located at any of nucleotides at positions 1 to 8 starting from the 5' terminus of the AS; and
preferably, the modified AS comprises a nucleotide sequence shown in any of SEQ ID NOs: 4-35 or a fragment thereof.

**3.** The oligonucleotide or the pharmaceutically acceptable salt thereof according to claim 1 or 2, wherein the 5'-phosphate mimic is selected from one or more of 5'-oxymethylphosphonate, 5'-vinylphosphonic acid (Vp), 5'-vinylphosphonate-2'-N-acetyl, or 5'-malonylphosphonate, and is more preferably one or more of 5'-vinylphosphonic acid (Vp) or 5'-vinylphosphonate-2'-N-acetyl, and is more preferably 5'-vinylphosphonic acid (Vp); and preferably, the 5'-phosphate mimic is modified at the 5' terminus of the AS.

**4.** The oligonucleotide or the pharmaceutically acceptable salt thereof according to any one of claims 1 to 3, wherein the oligonucleotide comprises at least one modified internucleotide linkage; and
preferably, the at least one modified internucleotide linkage is a phosphorothioate linkage.

**5.** The oligonucleotide or the pharmaceutically acceptable salt thereof according to any one of claims 1 to 4, wherein the oligonucleotide comprises a combination of SS and AS selected from the group consisting of:

(1) the SS shown in SEQ ID NO: 1 and the AS shown in SEQ ID NO: 3;
(2) the SS shown in SEQ ID NO: 1 and the AS shown in SEQ ID NO: 4;
(3) the SS shown in SEQ ID NO: 1 and the AS shown in SEQ ID NO: 5;
(4) the SS shown in SEQ ID NO: 1 and the AS shown in SEQ ID NO: 6;
(5) the SS shown in SEQ ID NO: 1 and the AS shown in SEQ ID NO: 7;
(6) the SS shown in SEQ ID NO: 1 and the AS shown in SEQ ID NO: 8;
(7) the SS shown in SEQ ID NO: 1 and the AS shown in SEQ ID NO: 9;
(8) the SS shown in SEQ ID NO: 1 and the AS shown in SEQ ID NO: 10;
(9) the SS shown in SEQ ID NO: 1 and the AS shown in SEQ ID NO: 11;
(10) the SS shown in SEQ ID NO: 1 and the AS shown in SEQ ID NO: 12;
(11) the SS shown in SEQ ID NO: 1 and the AS shown in SEQ ID NO: 13;
(12) the SS shown in SEQ ID NO: 1 and the AS shown in SEQ ID NO: 14;
(13) the SS shown in SEQ ID NO: 1 and the AS shown in SEQ ID NO: 15;
(14) the SS shown in SEQ ID NO: 1 and the AS shown in SEQ ID NO: 16;
(15) the SS shown in SEQ ID NO: 1 and the AS shown in SEQ ID NO: 17;
(16) the SS shown in SEQ ID NO: 1 and the AS shown in SEQ ID NO: 18;
(17) the SS shown in SEQ ID NO: 1 and the AS shown in SEQ ID NO: 19;
(18) the SS shown in SEQ ID NO: 1 and the AS shown in SEQ ID NO: 20;
(19) the SS shown in SEQ ID NO: 1 and the AS shown in SEQ ID NO: 21;
(20) the SS shown in SEQ ID NO: 1 and the AS shown in SEQ ID NO: 22;
(21) the SS shown in SEQ ID NO: 1 and the AS shown in SEQ ID NO: 23;
(22) the SS shown in SEQ ID NO: 1 and the AS shown in SEQ ID NO: 24;
(23) the SS shown in SEQ ID NO: 1 and the AS shown in SEQ ID NO: 25;
(24) the SS shown in SEQ ID NO: 1 and the AS shown in SEQ ID NO: 26;
(25) the SS shown in SEQ ID NO: 1 and the AS shown in SEQ ID NO: 27;
(26) the SS shown in SEQ ID NO: 1 and the AS shown in SEQ ID NO: 28;
(27) the SS shown in SEQ ID NO: 1 and the AS shown in SEQ ID NO: 29;
(28) the SS shown in SEQ ID NO: 1 and the AS shown in SEQ ID NO: 30;
(29) the SS shown in SEQ ID NO: 1 and the AS shown in SEQ ID NO: 31;
(30) the SS shown in SEQ ID NO: 1 and the AS shown in SEQ ID NO: 32;
(31) the SS shown in SEQ ID NO: 2 and the AS shown in SEQ ID NO: 33;
(32) the SS shown in SEQ ID NO: 1 and the AS shown in SEQ ID NO: 34; and
(33) the SS shown in SEQ ID NO: 1 and the AS shown in SEQ ID NO: 35.

**6.** The oligonucleotide or the pharmaceutically acceptable salt thereof according to any one of claims 1 to 5, wherein at least one nucleotide of the oligonucleotide is conjugated to one or more targeting ligands;

preferably, the targeting ligand is conjugated to the 3' terminus of the SS of the oligonucleotide;
preferably, the targeting ligand comprises a carbohydrate, an amino sugar, cholesterol, a polypeptide, or a lipid;
preferably, the targeting ligand comprises the amino sugar;
preferably, the targeting ligand comprises an N-acetylgalactosamine (GalNAc) moiety;
preferably, the GalNAc moiety is a monovalent GalNAc moiety, a divalent GalNAc moiety, a trivalent GalNAc moiety, or a tetravalent GalNAc moiety;
preferably, the targeting ligand has the following structure:

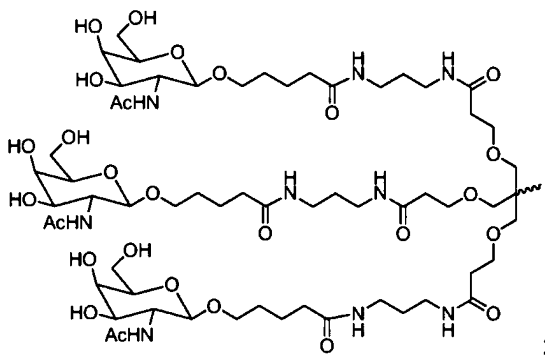
;

and

preferably, the targeting ligand is conjugated to the 3' terminus of the SS of the oligonucleotide through a linker to produce the following conjugate:

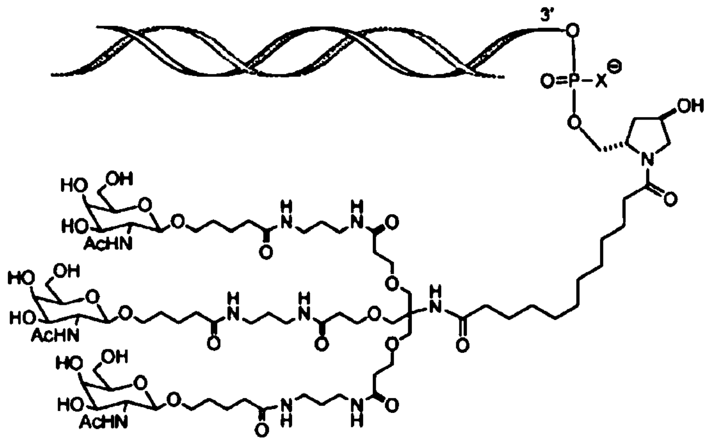

,

wherein X can be selected from S or O.

**7.** The oligonucleotide or the pharmaceutically acceptable salt thereof according to any one of claims 1 to 6, wherein the oligonucleotide comprises a combination of SS and AS selected from the group consisting of:

(1) the SS shown in SEQ ID NO: 36 and the AS shown in SEQ ID NO: 3;
(2) the SS shown in SEQ ID NO: 36 and the AS shown in SEQ ID NO: 4;
(3) the SS shown in SEQ ID NO: 36 and the AS shown in SEQ ID NO: 27;
(4) the SS shown in SEQ ID NO: 36 and the AS shown in SEQ ID NO: 28;
(5) the SS shown in SEQ ID NO: 36 and the AS shown in SEQ ID NO: 29;
(6) the SS shown in SEQ ID NO: 36 and the AS shown in SEQ ID NO: 30;
(7) the SS shown in SEQ ID NO: 36 and the AS shown in SEQ ID NO: 31;
(8) the SS shown in SEQ ID NO: 36 and the AS shown in SEQ ID NO: 32;
(9) the SS shown in SEQ ID NO: 37 and the AS shown in SEQ ID NO: 39;
(10) the SS shown in SEQ ID NO: 38 and the AS shown in SEQ ID NO: 33;
(11) the SS shown in SEQ ID NO: 36 and the AS shown in SEQ ID NO: 34; and
(12) the SS shown in SEQ ID NO: 36 and the AS shown in SEQ ID NO: 35.

**8.** A composition, comprising the oligonucleotide or the pharmaceutically acceptable salt thereof according to any one of claims 1 to 7, and optionally a pharmaceutically acceptable carrier.

**9.** Use of the oligonucleotide or the pharmaceutically acceptable salt thereof according to any one of claims 1 to 7 or the composition according to claim 8 in the manufacture of a medicament for reducing at least one of hepatitis B surface antigen (HBsAg), hepatitis B e antigen (HBeAg) and HBV DNA in serum of a subject.

**10.** Use of the oligonucleotide or the pharmaceutically acceptable salt thereof according to any one of claims 1 to 7 or the composition according to claim 8 in the manufacture of a medicament for treating HBV infection or hepatitis B and/or HBV-hepatitis D virus (HDV) coinfection;

preferably, the HBV infection is chronic HBV infection; and
preferably, the hepatitis B is chronic hepatitis B (CHB).

**11.** A method for treating HBV infection or hepatitis B and/or HBV-HDV coinfection, comprising administering a therapeutically effective amount of oligonucleotide or pharmaceutically acceptable salt thereof according to any one of claims 1 to 7 or the composition according to claim 8 to a subject in need thereof;

preferably, the HBV infection is chronic HBV infection; and
preferably, the hepatitis B is CHB.

**12.** The oligonucleotide or the pharmaceutically acceptable salt thereof according to any one of claims 1 to 7 or the composition according to claim 8, for use in the treatment of HBV infection or hepatitis B and/or HBV-HDV coinfection;

preferably, the HBV infection is chronic HBV infection; and
preferably, the hepatitis B is CHB.

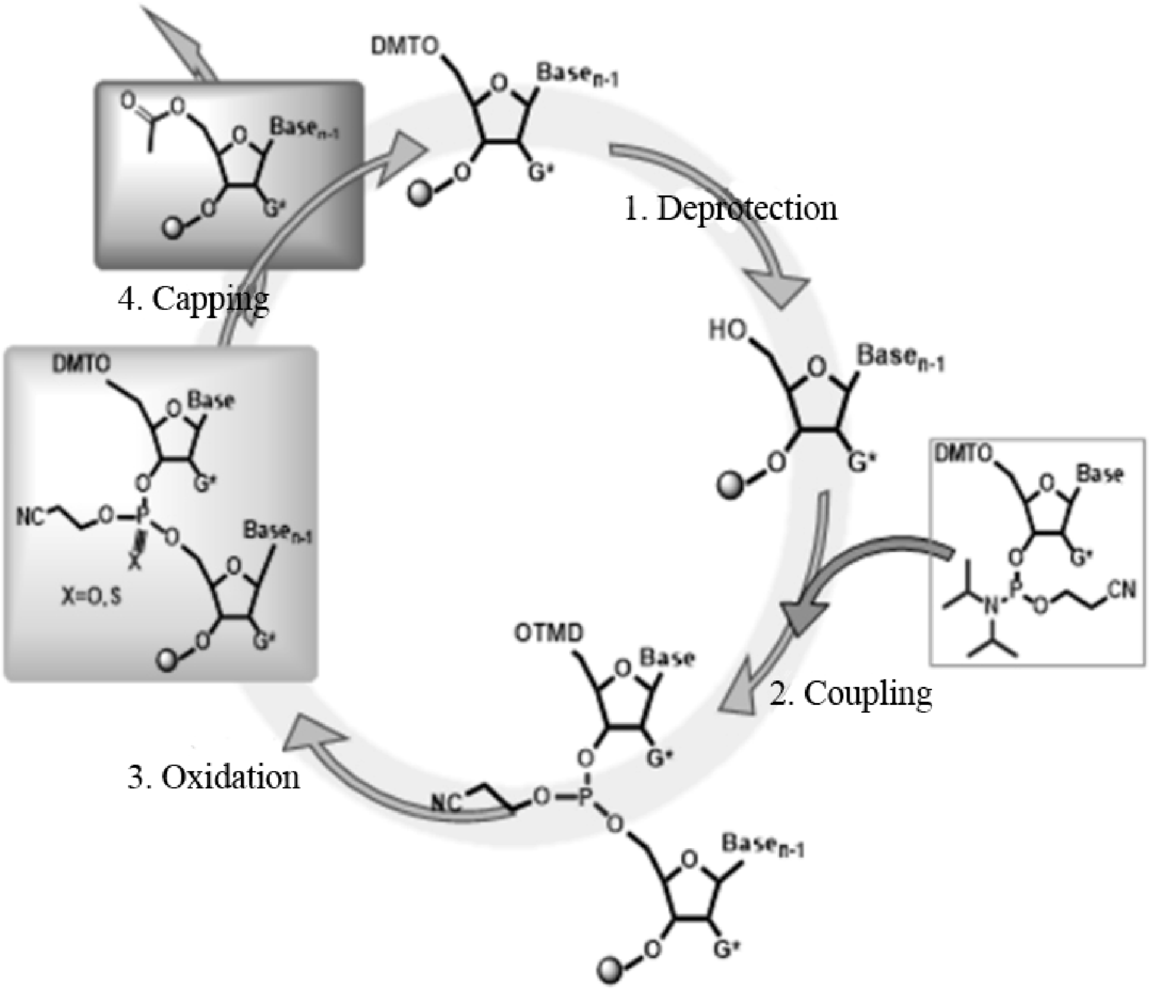


FIG. 1

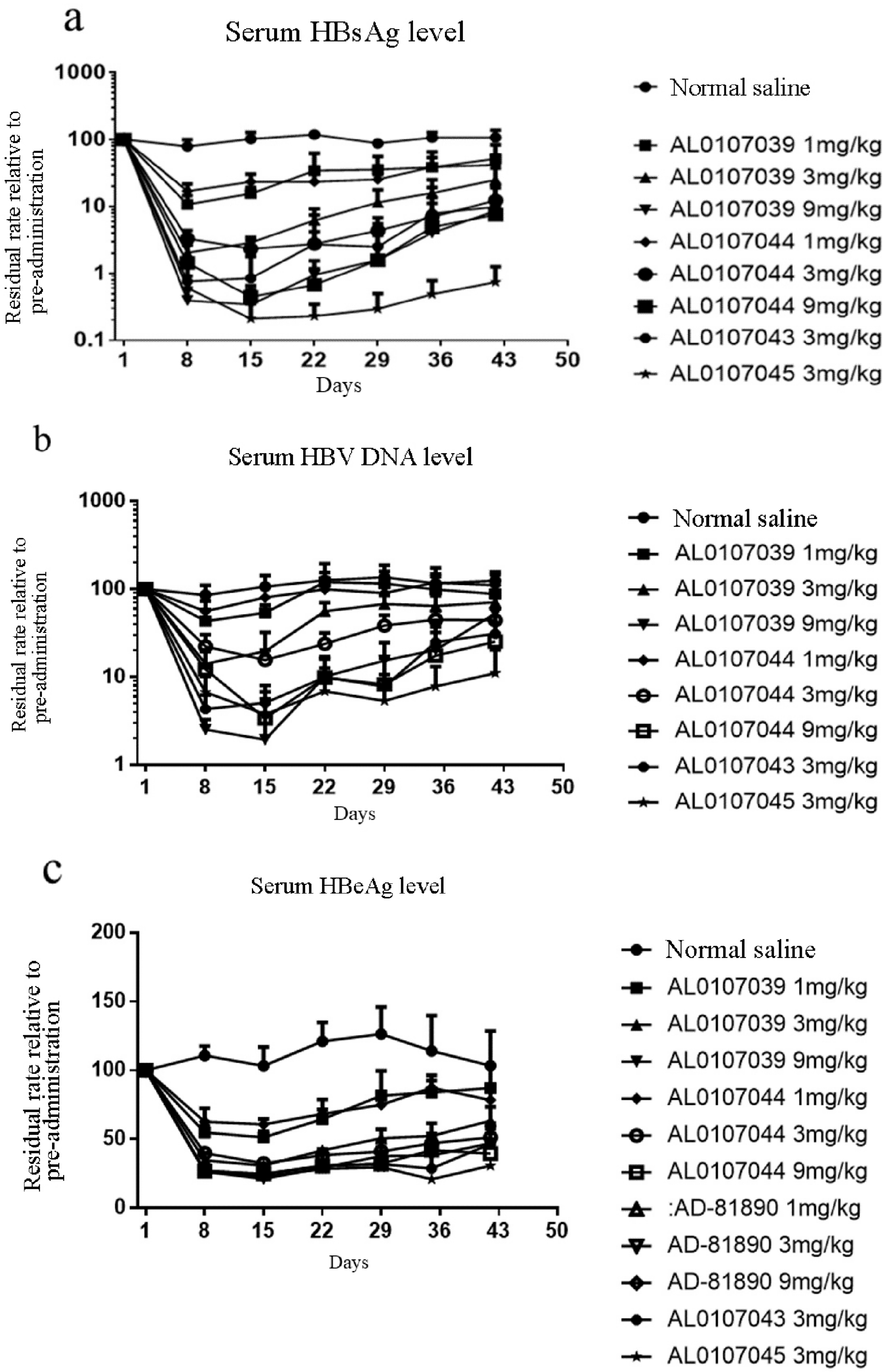
a
Serum HBsAg level
Residual rate relative to pre-administration
1000
100
10
1
0.1
1
8
15
22
29
36
43
50
Days
Normal saline
AL0107039 1mg/kg
AL0107039 3mg/kg
AL0107039 9mg/kg
AL0107044 1mg/kg
AL0107044 3mg/kg
AL0107044 9mg/kg
AL0107043 3mg/kg
AL0107045 3mg/kg
b
Serum HBV DNA level
Residual rate relative to pre-administration
1000
100
10
1
1
8
15
22
29
36
43
50
Days
Normal saline
AL0107039 1mg/kg
AL0107039 3mg/kg
AL0107039 9mg/kg
AL0107044 1mg/kg
AL0107044 3mg/kg
AL0107044 9mg/kg
AL0107043 3mg/kg
AL0107045 3mg/kg
c
Serum HBeAg level
Residual rate relative to pre-administration
200
150
100
50
0
1
8
15
22
29
36
43
50
Days
Normal saline
AL0107039 1mg/kg
AL0107039 3mg/kg
AL0107039 9mg/kg
AL0107044 1mg/kg
AL0107044 3mg/kg
AL0107044 9mg/kg
:AD-81890 1mg/kg
AD-81890 3mg/kg
AD-81890 9mg/kg
AL0107043 3mg/kg
AL0107045 3mg/kg

FIG. 2

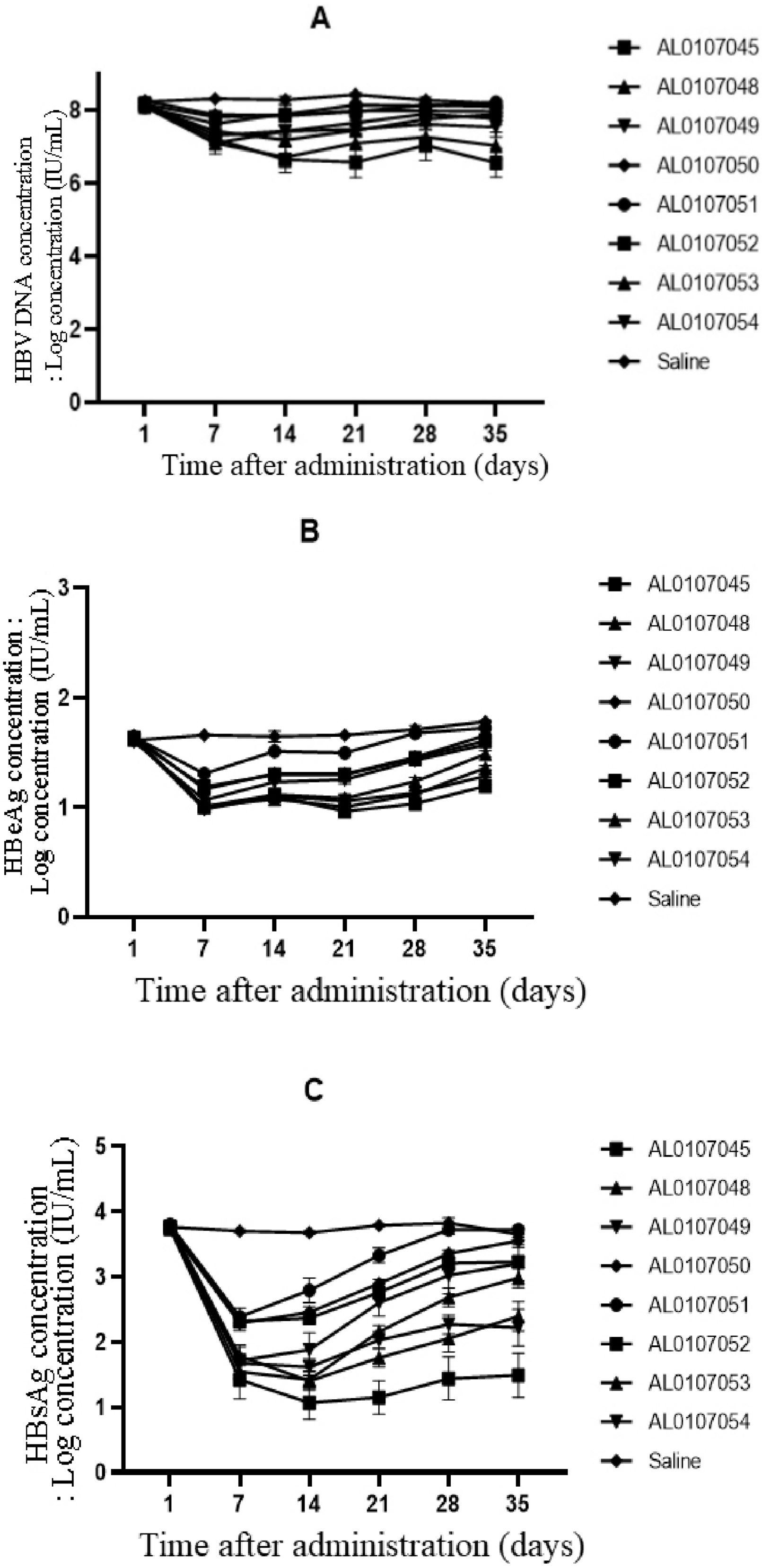
A
HBV DNA concentration
: Log concentration (IU/mL)
0
2
4
6
8
1
7
14
21
28
35
Time after administration (days)
AL0107045
AL0107048
AL0107049
AL0107050
AL0107051
AL0107052
AL0107053
AL0107054
Saline
B
HBeAg concentration :
Log concentration (IU/mL)
0
1
2
3
1
7
14
21
28
35
Time after administration (days)
AL0107045
AL0107048
AL0107049
AL0107050
AL0107051
AL0107052
AL0107053
AL0107054
Saline
C
HBsAg concentration
: Log concentration (IU/mL)
0
1
2
3
4
5
1
7
14
21
28
35
Time after administration (days)
AL0107045
AL0107048
AL0107049
AL0107050
AL0107051
AL0107052
AL0107053
AL0107054
Saline

FIG. 3

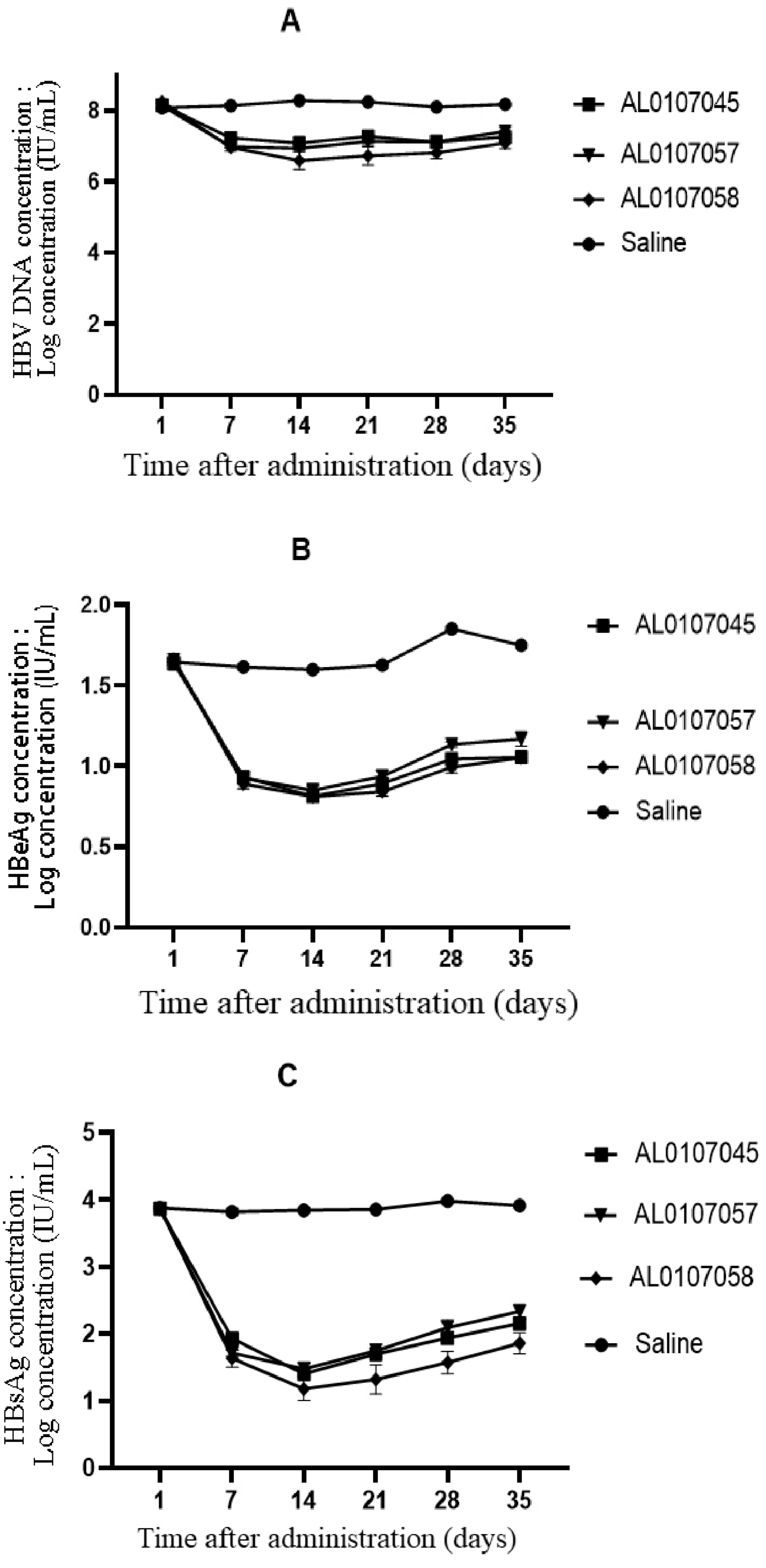
A
HBV DNA concentration : Log concentration (IU/mL)
0
2
4
6
8
1
7
14
21
28
35
Time after administration (days)
AL0107045
AL0107057
AL0107058
Saline
B
HBeAg concentration : Log concentration (IU/mL)
0.0
0.5
1.0
1.5
2.0
1
7
14
21
28
35
Time after administration (days)
AL0107045
AL0107057
AL0107058
Saline
C
HBsAg concentration : Log concentration (IU/mL)
0
1
2
3
4
5
1
7
14
21
28
35
Time after administration (days)
AL0107045
AL0107057
AL0107058
Saline

FIG. 4

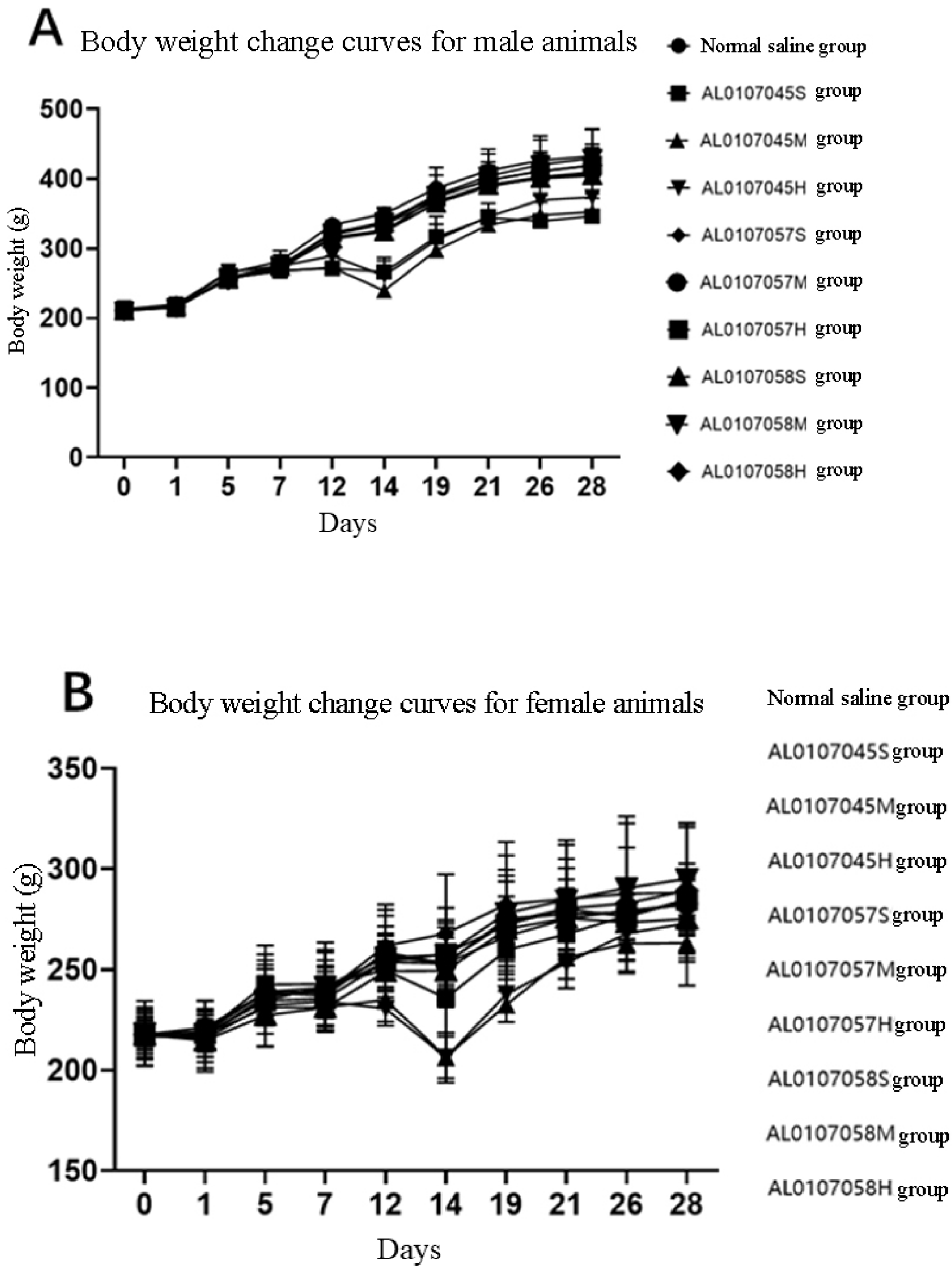
A
Body weight change curves for male animals
Normal saline group
AL0107045S group
AL0107045M group
AL0107045H group
AL0107057S group
AL0107057M group
AL0107057H group
AL0107058S group
AL0107058M group
AL0107058H group
Body weight (g)
500
400
300
200
100
0
0
1
5
7
12
14
19
21
26
28
Days
B
Body weight change curves for female animals
Normal saline group
AL0107045S group
AL0107045M group
AL0107045H group
AL0107057S group
AL0107057M group
AL0107057H group
AL0107058S group
AL0107058M group
AL0107058H group
Body weight (g)
350
300
250
200
150
0
1
5
7
12
14
19
21
26
28
Days

FIG. 5

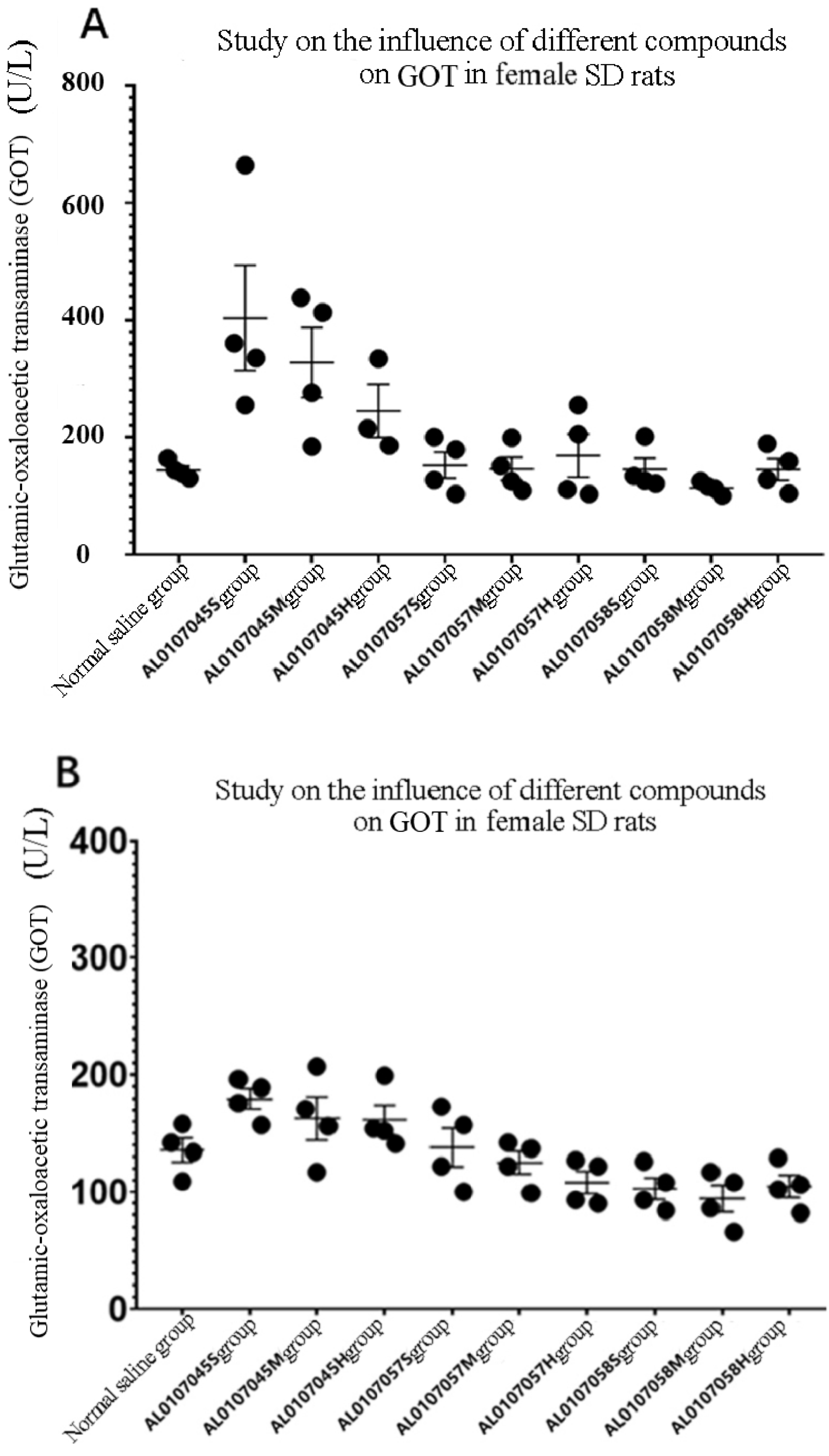
A
Study on the influence of different compounds
on GOT in female SD rats
Glutamic-oxaloacetic transaminase (GOT) (U/L)
800
600
400
200
0
Normal saline group
AL0107045Sgroup
AL0107045Mgroup
AL0107045Hgroup
AL0107057Sgroup
AL0107057Mgroup
AL0107057Hgroup
AL0107058Sgroup
AL0107058Mgroup
AL0107058Hgroup
B
Study on the influence of different compounds
on GOT in female SD rats
Glutamic-oxaloacetic transaminase (GOT) (U/L)
400
300
200
100
0
Normal saline group
AL0107045Sgroup
AL0107045Mgroup
AL0107045Hgroup
AL0107057Sgroup
AL0107057Mgroup
AL0107057Hgroup
AL0107058Sgroup
AL0107058Mgroup
AL0107058Hgroup

FIG. 6

## INTERNATIONAL SEARCH REPORT

International application No. **PCT/CN2024/122464**

**A. CLASSIFICATION OF SUBJECT MATTER**

C12N 15/113(2010.01)i； A61K 31/713(2006.01)i； A61P 31/20(2006.01)i； A61P 1/16(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, 超星读秀学术, Chaoxing Duxiu Scholar, 中国专利生物序列检索系统, China Patent Biological Sequence Search System, CNKI, 万方, Wanfang, ISI web of knowledge, Elsevier Science Direct, Springerlink, pubmed, genbank, ENA, EMBL, Uniport, STN, 百度, Baidu, 百度学术, Baidu Scholar: 肝炎病毒, B型, HBV, 乙肝, 乙型, sirna, 小干扰RNA, 修饰, 缀合, modification, 凯因, 序列blast, sequence BLAST

**C. DOCUMENTS CONSIDERED TO BE RELEVANT**

| Category* | Citation of document, with indication, where appropriate, of the relevant passages | Relevant to claim No. |
|---|---|---|
| A | CN 110846320 A (KYLONOVA (XIAMEN) BIOPHARMA CO., LTD.) 28 February 2020 (2020-02-28)<br>claims 1-12, and description, paragraphs 25, 26, 30, 41 and 42 | 1-12 |
| A | CN 116162622 A (SAINUORUI BIOMEDICAL TECHNOLOGY (WUXI) CO., LTD.) 26 May 2023 (2023-05-26)<br>claims 1-34, and description, paragraphs 183-184, 277, 283, 288, 299 and 303 | 1-12 |
| A | CN 103635576 A (ARROWHEAD RESEARCH CORPORATION) 12 March 2014 (2014-03-12)<br>claims 1-20 | 1-12 |
| A | CN 112673103 A (ALNYLAM PHARMACEUTICALS, INC.) 16 April 2021 (2021-04-16)<br>claims 1-70 | 1-12 |
| A | CN 114846140 A (F. HOFFMANN-LA ROCHE AG) 02 August 2022 (2022-08-02)<br>claims 1-159 | 1-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 January 2025** | **20 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/122464**

**C. DOCUMENTS CONSIDERED TO BE RELEVANT**

| Category* | Citation of document, with indication, where appropriate, of the relevant passages | Relevant to claim No. |
|---|---|---|
| A | WO 2022206946 A1 (TUOJIE BIOTECH (SHANGHAI) CO., LTD.) 06 October 2022 (2022-10-06) claims 1-21 | 1-12 |
| A | EP 4166144 A1 (CHIA TAI TIANQING PHARMACEUTICAL GROUP CO., LTD. et al.) 19 April 2023 (2023-04-19) claims 1-20 | 1-12 |
| A | CN 114507663 A (ZHEJIANG BAILAATU MEDICINE SCIENCE AND TECHNOLOGY LIMITED COMPANY) 17 May 2022 (2022-05-17) claims 1-10 | 1-12 |
| A | WO 2021110148 A1 (MEDSHINE DISCOVERY INC.) 10 June 2021 (2021-06-10) claims 1-21 | 1-12 |

Form PCT/ISA/210 (second sheet) (July 2022)

**INTERNATIONAL SEARCH REPORT**

International application No.
**PCT/CN2024/122464**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/122464**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

Claim 11 relates to a method for treating a living human or animal body, and therefore does not comply with PCT Rule 39.1(iv). In the present report, a search is carried out on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/122464**

| Patent document cited in search report | | | Publication date (day/month/year) | Patent family member(s) | | | Publication date (day/month/year) |
|---|---|---|---|---|---|---|---|
| CN | 110846320 | A | 28 February 2020 | JP | 2022540363 | A | 15 September 2022 |
| | | | | JP | 7383308 | B2 | 20 November 2023 |
| | | | | US | 2024368600 | A1 | 07 November 2024 |
| | | | | WO | 2020259497 | A1 | 30 December 2020 |
| | | | | KR | 20220035398 | A | 22 March 2022 |
| | | | | KR | 102713731 | B1 | 04 October 2024 |
| | | | | US | 2023374513 | A1 | 23 November 2023 |
| | | | | US | 2023110095 | A1 | 13 April 2023 |
| | | | | US | 11840691 | B2 | 12 December 2023 |
| | | | | EP | 3992291 | A1 | 04 May 2022 |
| | | | | EP | 3992291 | A4 | 02 August 2023 |
| | | | | AU | 2020305793 | A1 | 17 February 2022 |
| | | | | AU | 2020305793 | B2 | 18 April 2024 |
| | | | | TW | 202043474 | A | 01 December 2020 |
| | | | | TWI | 750712 | B | 21 December 2021 |
| CN | 116162622 | A | 26 May 2023 | | None | | |
| CN | 103635576 | A | 12 March 2014 | USRE | 48345 | E | 08 December 2020 |
| | | | | WO | 2013003520 | A1 | 03 January 2013 |
| | | | | AU | 2022202365 | A1 | 28 April 2022 |
| | | | | ZA | 201307914 | B | 28 January 2015 |
| | | | | ES | 2690538 | T3 | 21 November 2018 |
| | | | | IL | 284578 | A | 31 August 2021 |
| | | | | IL | 230226 | B | 31 December 2019 |
| | | | | TW | 202244278 | A | 16 November 2022 |
| | | | | KR | 20190111147 | A | 01 October 2019 |
| | | | | KR | 102167524 | B1 | 20 October 2020 |
| | | | | HK | 1193633 | A1 | 26 September 2014 |
| | | | | TW | 201311716 | A | 16 March 2013 |
| | | | | TWI | 659040 | B | 11 May 2019 |
| | | | | PT | 2726613 | T | 26 October 2018 |
| | | | | EP | 3901263 | A1 | 27 October 2021 |
| | | | | AU | 2017210563 | A1 | 24 August 2017 |
| | | | | AU | 2017210563 | B2 | 10 October 2019 |
| | | | | IL | 270877 | A | 30 January 2020 |
| | | | | IL | 270877 | B | 31 October 2021 |
| | | | | MX | 2013013556 | A | 27 February 2014 |
| | | | | MX | 342731 | B | 11 October 2016 |
| | | | | AU | 2012275355 | A1 | 31 October 2013 |
| | | | | AU | 2012275355 | A2 | 13 February 2014 |
| | | | | AU | 2012275355 | B2 | 11 May 2017 |
| | | | | PL | 2726613 | T3 | 28 February 2019 |
| | | | | DK | 2726613 | T3 | 03 December 2018 |
| | | | | NZ | 617756 | A | 24 April 2015 |
| | | | | JP | 2021191274 | A | 16 December 2021 |
| | | | | KR | 20140028021 | A | 07 March 2014 |
| | | | | KR | 102025295 | B1 | 26 September 2019 |
| | | | | EA | 201391394 | A1 | 30 May 2014 |
| | | | | EA | 024762 | B1 | 31 October 2016 |
| | | | | EA | 024762 | B9 | 28 April 2017 |
| | | | | TW | 201827594 | A | 01 August 2018 |

Form PCT/ISA/210 (patent family annex) (July 2022)

**INTERNATIONAL SEARCH REPORT**
**Information on patent family members**

International application No.
**PCT/CN2024/122464**

| Patent document cited in search report | | | Publication date (day/month/year) | Patent family member(s) | | | Publication date (day/month/year) |
|---|---|---|---|---|---|---|---|
| | | | | TWI | 695066 | B | 01 June 2020 |
| | | | | KR | 20200122397 | A | 27 October 2020 |
| | | | | KR | 102434346 | B1 | 18 August 2022 |
| | | | | SI | 2726613 | T1 | 30 October 2018 |
| | | | | CA | 2833778 | A1 | 03 January 2013 |
| | | | | CA | 2833778 | C | 28 September 2021 |
| | | | | KR | 20210110900 | A | 09 September 2021 |
| | | | | KR | 102434357 | B1 | 18 August 2022 |
| | | | | EP | 3418386 | A2 | 26 December 2018 |
| | | | | EP | 3418386 | A3 | 24 April 2019 |
| | | | | EP | 3418386 | B1 | 07 April 2021 |
| | | | | TW | 202424196 | A | 16 June 2024 |
| | | | | TW | 202102673 | A | 16 January 2021 |
| | | | | TWI | 786401 | B | 11 December 2022 |
| | | | | KR | 20220119522 | A | 29 August 2022 |
| | | | | KR | 102554783 | B1 | 11 July 2023 |
| | | | | SG | 194751 | A1 | 30 December 2013 |
| | | | | US | 2013005793 | A1 | 03 January 2013 |
| | | | | US | 8809293 | B2 | 19 August 2014 |
| | | | | EA | 201690497 | A2 | 31 August 2016 |
| | | | | EA | 201690497 | A3 | 30 December 2016 |
| | | | | EA | 035756 | B1 | 05 August 2020 |
| | | | | JP | 2019141077 | A | 29 August 2019 |
| | | | | JP | 6959293 | B2 | 02 November 2021 |
| | | | | HRP | 20181564 | T1 | 30 November 2018 |
| | | | | EA | 202090994 | A1 | 31 August 2020 |
| | | | | EA | 202191537 | A1 | 31 January 2022 |
| | | | | EP | 2726613 | A1 | 07 May 2014 |
| | | | | EP | 2726613 | A4 | 10 December 2014 |
| | | | | EP | 2726613 | B1 | 08 August 2018 |
| | | | | AU | 2022206748 | A1 | 18 August 2022 |
| | | | | BR | 112013031021 | A2 | 09 October 2018 |
| | | | | MX | 2020011807 | A | 07 December 2020 |
| | | | | JP | 2017169568 | A | 28 September 2017 |
| | | | | JP | 2023179497 | A | 19 December 2023 |
| | | | | CA | 3064103 | A1 | 03 January 2013 |
| | | | | CA | 3064103 | C | 07 March 2023 |
| | | | | JP | 2014527401 | A | 16 October 2014 |
| | | | | JP | 6165723 | B2 | 19 July 2017 |
| | | | | AU | 2019240625 | A1 | 17 October 2019 |
| | | | | AU | 2019240625 | B2 | 21 April 2022 |
| CN | 112673103 | A | 16 April 2021 | JP | 2021533767 | A | 09 December 2021 |
| | | | | US | 2023128522 | A1 | 27 April 2023 |
| | | | | UA | 127745 | C2 | 20 December 2023 |
| | | | | CA | 3106701 | A1 | 20 February 2020 |
| | | | | US | 2021332365 | A1 | 28 October 2021 |
| | | | | US | 11492623 | B2 | 08 November 2022 |
| | | | | WO | 2020036862 | A1 | 20 February 2020 |
| | | | | WO | 2020036862 | A9 | 11 March 2021 |
| | | | | SG | 11202100715 | WA | 25 February 2021 |

Form PCT/ISA/210 (patent family annex) (July 2022)

**INTERNATIONAL SEARCH REPORT**
**Information on patent family members**

International application No.
**PCT/CN2024/122464**

| Patent document cited in search report | | | Publication date (day/month/year) | Patent family member(s) | | | Publication date (day/month/year) |
|---|---|---|---|---|---|---|---|
| | | | | BR | 112021001613 | A2 | 04 May 2021 |
| | | | | EP | 3837366 | A1 | 23 June 2021 |
| | | | | EA | 202190528 | A1 | 23 April 2021 |
| | | | | AU | 2019321375 | A1 | 11 March 2021 |
| | | | | PH | 12021550287 | A1 | 27 September 2021 |
| | | | | AR | 114551 | A1 | 16 September 2020 |
| | | | | IL | 280559 | A | 25 March 2021 |
| | | | | TW | 202023574 | A | 01 July 2020 |
| | | | | JOP | 20210007 | A1 | 12 January 2021 |
| | | | | MX | 2021001056 | A | 12 April 2021 |
| | | | | KR | 20210043647 | A | 21 April 2021 |
| CN | 114846140 | A | 02 August 2022 | AU | 2020410993 | A1 | 16 June 2022 |
| | | | | MX | 2022007908 | A | 21 July 2022 |
| | | | | US | 2023119360 | A1 | 20 April 2023 |
| | | | | JP | 2023509870 | A | 10 March 2023 |
| | | | | WO | 2021130266 | A1 | 01 July 2021 |
| | | | | EP | 4081639 | A1 | 02 November 2022 |
| | | | | CA | 3163490 | A1 | 01 July 2021 |
| | | | | TW | 202137987 | A | 16 October 2021 |
| WO | 2022206946 | A1 | 06 October 2022 | TW | 202302851 | A | 16 January 2023 |
| EP | 4166144 | A1 | 19 April 2023 | JP | 2023528966 | A | 06 July 2023 |
| | | | | EP | 4166144 | A4 | 10 July 2024 |
| | | | | CA | 3186763 | A1 | 16 December 2021 |
| | | | | KR | 20230022892 | A | 16 February 2023 |
| CN | 114507663 | A | 17 May 2022 | None | | | |
| WO | 2021110148 | A1 | 10 June 2021 | US | 2023220386 | A1 | 13 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023112745419
- CN 104717982 B
- US 20130178612 A1
- US 2015100197 A1
- GB 149252010 A


### Non-patent literature cited in the description


- **NICOLINI LA et al.** *Int. J. Environ. Res. Public Health*, 2019, vol. 16, 3307
- **SUAREZ AAR et al.** *Liver International.*, 2021, vol. 41 (1), 15-23
- **FIRE** ; **ANDREW et al.** *nature*, 1998, vol. 391 (6669), 806-811
- *J. Med. Chem.*, 2018, vol. 61, 734-744